# EUROPEAN PATENT APPLICATION

(11) **EP 3 587 454 A1**
(43) Date of publication of application: **01.01.2020**
(21) Application number: 18180026.9
(22) Date of filing: 27.06.2018
(51) Int. Cl.: C07K 16/30, A61K 39/00, A61K 39/395, A61K 31/00, C07K 14/725, A61P 35/00

(54) **CHIMERIC ANTIGEN RECEPTORS THAT BIND TO PROSTATE SPECIFIC MEMBRANE ANTIGEN**

(71) Applicant: Albert-Ludwigs-Universität Freiburg, 79098 Freiburg (DE)
(72) Inventor: The designation of the inventor has not yet been filed
(74) Representative: Keller, Günter

(57) **Abstract**

The present invention relates to a novel chimeric antigen receptor (CAR) comprising an antigen-binding fragment which binds specifically to PSMA antigen, and a method of manufacturing high-quality CAR T cell products by transfection and/or transduction of T cells therewith, which allows to eradicate tumors in vivo alone or in combination with pharmaceutical drugs, such chemotherapies, biopharmaceutical drugs, such as antibodies, or small-molecule drugs, such as protein kinase inhibitors.

## Description

The present invention relates to chimeric antigen receptors that bind to tumor antigens whereby the antigen is the prostate specific membrane antigen (PSMA). The chimeric antigen receptors (in the following CAR) are brought into immune cells (T cells) which then specifically react with tumor cells expressing PSMA which leads to the elimination of the tumor cells. The constructs of the present invention contain two major parts. On the one hand the antigen-binding region which specifically binds to the prostate specific membrane antigen (PSMA) and on the other hand co-stimulatory domains derived from a receptor of an immune cell responsible for signal transduction and activation of the immune cell.

Prostate cancer remains the second-most frequently diagnosed cancer among men worldwide with estimated 1.1 million new cases per year. Moreover, with expected 307,000 deaths, it represents the fifth leading cause of cancer deaths. Whereas primary tumors can successfully be treated, there is no curative treatment for advanced stages. Therefore, new therapeutic options are urgently needed.

The prostate specific membrane antigen (PSMA) is the best characterized antigen in prostate cancer for antibody-based diagnostic and therapeutic intervention. This protein is also known as glutamate carboxypeptidase II (EC 3.4.17.21), N-acetyl-linked acidic dipeptidase I (NAALADase), or folate hydrolase. PSMA is a type II membrane glycoprotein consisting of 750 amino acids (aa) with a small intracellular domain of 19 aa, a transmembrane domain of 24 aa, and a large extracellular domain of 707 aa. The extracellular domain folds into three distinct domains: the protease domain (aa 57-116 and 352-590), the apical domain (aa 117-351), and the C-terminal domain (aa 591-750). It shows a high structural similarity and identity to the human transferrin receptor 1. PSMA is highly restricted to the surface of prostate cancer cells, is present on all tumor stages, and shows an enhanced expression in androgen-independent and metastatic disease. PSMA is not secreted into the extracellular space and undergoes constitutive internalization, which is enhanced by binding of PSMA-specific antibodies. These characteristics make it an ideal candidate for the targeted treatment of advanced prostate cancer. Moreover, PSMA was also found to be expressed in the neovascular endothelium of virtually all solid tumor types without expression in normal vascular endothelium. It is therefore considered to be a unique antiangiogenic target.

Monoclonal antibodies (mAbs) are highly specific and versatile tools for cell targeting. In the last decades, they have attracted high interest in medical research and have become the most rapidly expanding class of pharmaceuticals for treating a variety of human diseases including cancer. Antibody 7E11 was the first published PSMA-specific mAb and was found to bind to the N-terminus (MWNLLH) of the intracellular domain of PSMA. The In-labeled form of 7E11 (ProstaScint, Cytogen, Philadelphia, PA) has received approval from the U.S. Food and Drug Administration (FDA) for the detection and imaging of metastatic prostate cancer in soft tissues. However, because the antibody binds an intracellular epitope, 7E11 is not capable to bind to viable cells. Positive signals in the *in vivo* imaging with ProstaScint had to be traced back to the detection of dead or dying cells within the tumor masses. Therefore, a new class of anti-PSMA mAbs was generated, which specifically bind to extracellular epitopes of PSMA.

EP 1 883 698 discloses three different mAbs, 3/A12, 3/E7, 3/F11, which show a strong and specific extracellular binding to PSMA on the surface of prostate cancer cells and tissue specimen. In direct comparison with mAb J591, a clinically validated antibody for radioimmunotherapy (PMID:18552139; PMID:24135437; PMID:25771365; PMID: 26175541), the mAb 3/F11 showed higher binding to PSMA expressing C4-2 prostate cancer target cells (K_{d} [defined as mean half-maximal saturation concentration] of 3/F11 = 9 nM; K_{d} of J591 = 16 nM). Moreover, competitive binding studies demonstrated that mAb 3/F11 binds to a different extracellular PSMA epitope than J591 (PMID: 19938014). In an immunohistological study on a panel of human normal tissues, no binding of the 3/F11 mAb to PSMA-negative tissues (adrenal, bone marrow, cerebellum, cerebrum, pituitary, colon, esophagus, heart, kidney, liver, lung, mesothelial cells of the pericardium, nerve, ovary, pancreas, skeletal muscle, skin, spleen, stomach, testis, thymus, thyroid, tonsil, and uterus) was detected. Only binding to secretory cells of the salivary glands and to duodenal brush border cells was observed, which are known to express PSMA (PMID:19938014). The mAb 3/F11 showed a moderate immunoreactivity on acinar secretory epithelial cells of all tested normal prostate tissues. A more intense and extensive staining was noticed in nearly all epithelial cells of adenocarcinomas as well as in lymph node metastases. No immunohistological staining on frozen sections of breast specimen was detected. In contrast and in accordance with other published data, staining of the mammary ductal epithelium was detected with mAb J591. Since PSMA expression in the breast tissue was neither detected by PCR nor by Western blotting, this is evidence that mAb J591 cross-reacts with another antigen.

The single-chain variable fragment (scFv) D7 (as disclosed in EP 1 883 698 B1) was generated by phage display technique from the mAb 3/F11. The most important fragments for the specificity of the anti-PSMA scFv are the V_{L} and the V_{H} parts, which are preferably linked with a polyglycin linker.-D7 bound to PSMA-expressing C4-2 cells with a K_{d} of about 18 nM, and preincubation with the parental mAb 3/F11 fully inhibited the binding activity. This proved that the scFv D7 binds to the same PSMA epitope as 3/F11. The scFv D7 and a humanized version thereof were successfully used for the construction of Pseudomonas Exotoxin A (PE) based immunotoxins, which showed high and specific cytotoxicity against PSMA-expressing prostate cancer cells and *in vivo* antitumor activity in mice bearing prostate tumors.

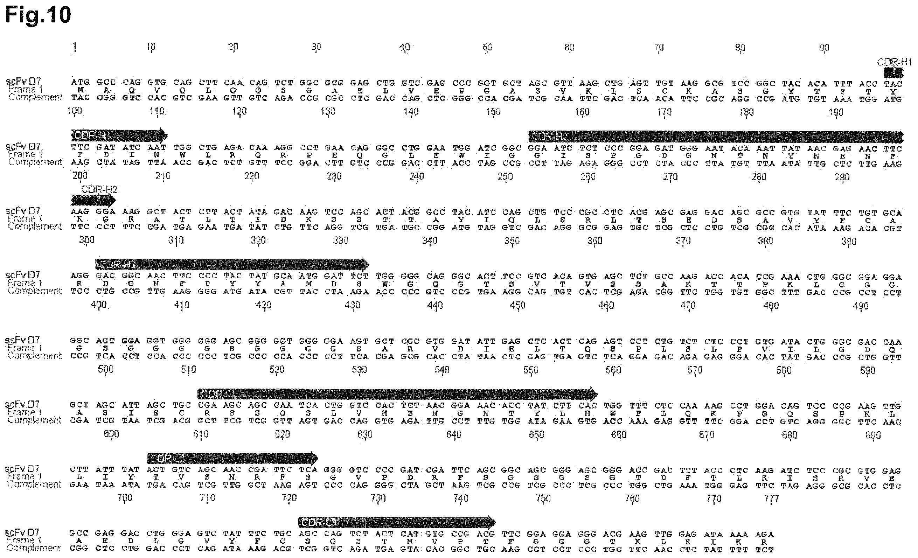

In vivo experiments, as disclosed herein, were performed in the mouse model and the sequences, as disclosed in Figure 10, were used.

The amino acid sequence of the antigen-binding fragment D7 (Figure 10; SEQ ID NO: 1) and the nucleic acid sequence (SEQ ID NO:8), including complementary strand coding for said construct (SEQ ID NO:9), is provided. Moreover, the highly important parts of the antigen-binding fragment, namely the CDRs: (CDR-H1 (SEQ ID NO:2), CDR-H2 (SEQ ID NO:3), CDR-H3 (SEQ ID NO:4), CDR-L1 (SEQ ID NO:5), CDR-L2 (SEQ ID NO:6) and CDR-L3 (SEQ ID NO:7)) are shown and highlighted by grey arrows.

Humanization of murine antibodies involves the transfer of beneficial properties (e.g. antigen-specific binding, avoidance of off-target effects by non-crossreactivity with other antigens) from one antibody to another to reduce immunogenicity. Humanization is usually necessary for human use as patients typically respond with an immune reaction against non-human antibodies that can lead to ineffectiveness of treatment and, in the worst-case scenario, to a life-threatening situation. A humanized construct can be derived from the sequence of the antigen-binding fragment shown in Figure 10. The CDR regions structurally define the paratope, that is, the contact site of the antigen-binding fragment with the antigen. The remainder of the sequence codes for the framework regions, which form the scaffold of the paratope. For the humanization process (e.g. by in silico modeling), the framework sequence is first compared with other antigen-binding sequences derived from humans. Usually a human sequence (acceptor framework) is selected which has the highest similarity with the framework sequence shown in Figure 10. The CDR regions are grafted into the human acceptor framework to eliminate amino acid sequences which may cause undesired human anti-mouse-antibody (HAMA) immune reactions. Substitutions at potentially critical positions (e.g. amino acids responsible for folding the paratope or the VH-VL interface) are analyzed for prospective back mutations. Even in the CDR sequences exceptional modifications of amino acids may be made to avoid immunogenicity, to ensure the right folding of the paratope, and to maintain the antigen-specific binding.

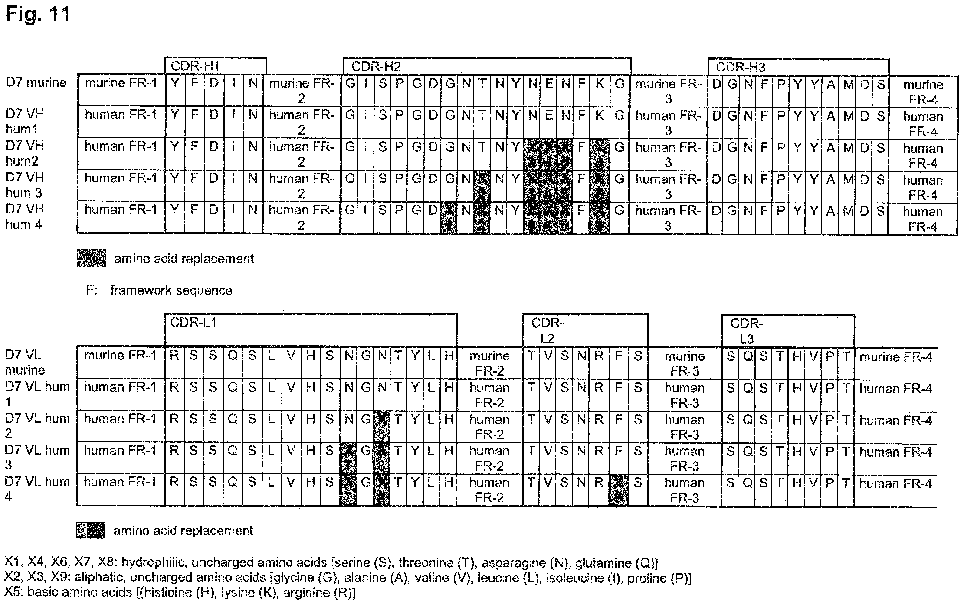

The results of the humanization experiments are shown in Figure 11. It turned out that the CDR-H1, CDR-H3 and CDR-L3 should be maintained without any amendment. In the CDR-L2 one amino acid can be replaced which is indicated as X9. X9 may have the meaning of an aliphatic, uncharged amino acid which can be glycine, alanine, valine, lysine, isolysine and/or proline.

In CDR-L1 two amino acids can be replaced which are designated as X7 and X8. Those amino acids can be hydrophilic, uncharged amino acids such as serine, threonine, asparagine and/or glutamine.

The highest flexibility seems to have CDR-H2 wherein up to six amino acids can be replaced. Those amino acids are designated as X1, X2, X3, X4, X5 and X6. The amino acids used for replacement in the humanized antibodies or antigen-binding fragments have the following meaning:
X1, X4, X6, X7, X8: hydrophilic, uncharged amino acids [serine (S), threonine (T), asparagine (N), glutamine (Q)]
X2, X3, X9: aliphatic, uncharged amino acids [glycine (G), alanine (A), valine (V), leucine (L), isoleucine (I), proline (P)]
X5: basic amino acids [histidine (H), lysine (K), arginine (R)].

In recent years, adoptive immune cell therapy has been introduced as a novel concept to treat different cancers by redirecting the immune system to eliminate the tumor cells. One of the most successful concepts is based on the genetic engineering of T cells to express chimeric antigen receptors (CARs) that bind tumor antigens or tumor-associated antigens in a human leukocyte antigen (HLA)-independent manner. CD19 targeting CAR T cells have been successfully used to treat B cell acute lymphoblastic leukemia (B-ALL), with >90% of patients going into complete remission in several clinical trials. Based on this success, more than 200 clinical trials have been initiated to treat mostly hematological malignancies. For solid tumors, however, the potency of CAR T cell therapy seems rather low to date. The main reason for this failure seems to be the tumor microenvironment (TME), which is the cellular environment in which the tumor exists. It includes various kinds of immune cells, fibroblasts, the extracellular matrix (ECM) as well as the surrounding blood vessels. Many mechanisms that describe restriction of cytotoxic T cell activity in the TME have been described, including the activation of PD-1 based T cell immune checkpoint inhibition. Overcoming these restrictions, combined with T cell checkpoint antagonists, will help to improve anti-tumor activity in the TME.

The tumor eradication, as used in the present invention, requires an adequate survival and intratumoral activation of tumor antigen-specific T cells. To meet these requirements T cells must be given appropriate activating signals at the time of antigen-priming and stimulation. The chimeric antigen receptors of the present invention combine therefore an antigen-binding fragment as part of the receptor on T cells. The antigen-binding fragment binds to the specific antigen (here PSMA) to which the T cells should bind. Moreover, the receptors contain sequences from CD28 and 4-1BB, respectively, as co-stimulatory signaling domains. It has been shown that the addition of CD28 sequences, or other co-stimulatory signaling domains, to CD3ζ chain-based receptors increases antigen-induced secretion of interleukin-2 and in vitro T cell expansion. In the present case the signaling domain consists of the CD3ζ domain and either the intracellular CD28 or the 4-1BB domain.

In general, the design of a CAR can vary and meanwhile several generations of CARs are known. The main components of a CAR system are the CD3ζ intracellular domain of the T cell receptor (TCR) complex, the transmembrane domain, the hinge region and the antigen-binding part. In the design of a CAR, the antigen-binding domain is linked to a hinge region, which is also called a spacer region, the transmembrane domain, and a cytoplasmic domain. Those parts are responsible for the position of the CAR, the attachment in T cell membrane, and intracellular signaling. Besides this structural rule in the CAR design the morphological characteristics of the hinge region, such as their length and sequence, are important for an efficient targeting. The intracellular domain acts as a signal transducer. The cytoplasmic segment of the CD3ζ plays the principal rule due to different functions in activated T cells and the resting ones. However, this cytoplasmic part cannot activate the resting T cells alone. Therefore, there is a need of at least a secondary signal for the full activation of T cells. In the present invention, preferably 4-1BB or CD28 co-stimulatory domains were used. Other co-stimulatory domains, such as co-stimulatory domains derived e.g. from CD27, ICOS and OX40, can be used alternatively.

For the treatment of human patients, T cells have to be enriched from the individual patient's peripheral blood (autologous setting) or provided by a donor (allogeneic setting). This can be done for example by leukapheresis. The enriched T cells are then transfected or transduced with a suitable vector comprising the genetic information for the essential fragments of the CAR.

The genetic information coding for the CAR is inserted into a suitable vector. A gold standard for transduction of primary T cells are presently considered lentiviral vectors which seem to be a valid alternative to simpler retroviral vectors. As a further alternative to lentiviral vectors the information can be introduced into the T cells with the help of transposons or plasmids. An alternative to both viral and non-viral delivery are the recently described gene editing tools, designated as CRISPR/Cas or other designer nucleases, such as transcription activator-like effector nucleases (TALENs). This technology platforms offer the possibility to target virtually any genomic site in a targeted manner. In the case of CRISPR/Cas, the editing complex comprises a Cas nuclease and a guide RNA, usually composed of a CRISPR RNA (crRNA) and a transacting crRNA. Upon hybridization of the guide RNA to the target sequence, Cas9 (or another Cas nuclease, such as e.g. Cpf1/Cas12a) generates a double-strand break, which can be repaired by non-homologous end joining (NHEJ), an event that can result in a loss of function of the genomic locus. In the presence of a suitable donor DNA, by a mechanism of homology-directed repair (HDR), an exogenous sequence (CAR sequence) can be introduced into the targeted locus. This can be exploited to deliver CAR expression cassette in a desired genomic locus that does not interfere with gene function and therefore minimizing the genotoxic effects experienced with integrating viral vectors. The nucleic acid sequence coding for the CARs of the present invention is preferably optimized for the human codon usage. Particularly preferred embodiments are SE ID NO:10 coding for the CAR28 construct and SEQ ID NO:11 coding for the CAR41 construct.

The chimeric antigen receptors disclosed herein can be used for the treatment of diseases which are related to the expression of PSMA. PSMA is expressed in tumor cells derived from prostatic cancer. There are several stages of prostatic cancer known, but it seems that PSMA is one of the markers best suited for the treatment of prostate cancer. The term "prostate cancer" comprises all forms of prostate cancer cells either derived from a primary tumor or from a metastatic tumor or from circulating tumor cells. In a particularly preferred embodiment the chimeric antigen receptors of the present invention are used against the neovascularization of solid tumors expressing PSMA.

In a further embodiment of the present invention the chimeric antigen receptors disclosed herein are used in combination with a cytotoxic agent. Cytotoxic agents as used for the treatment of prostate cancer are known. Preferably such substances comprise taxol derivatives, 5-fluorouracil, cyclophosphamide, mitoxantrone, docetaxel, cabazitaxel and etoposide. The following drugs are approved for prostate cancer and preferably used: Abiraterone Acetate, Apalutamide, Bicalutamide, Cabazitaxel, Casodex (Bicalutamide), Degarelix, Docetaxel, Eligard (Leuprolide Acetate), Enzalutamide, Erleada (Apalutamide), Firmagon (Degarelix), Flutamide, Goserelin Acetate, Jevtana (Cabazitaxel), Leuprolide Acetate, Lupron Depot (Leuprolide Acetate), Mitoxantrone Hydrochloride, Nilandron (Nilutamide), Nilutamide, Provenge (Sipuleucel-T), Radium 223 Dichloride, Sipuleucel-T, Taxotere (Docetaxel), Xofigo (Radium 223 Dichloride), Xtandi (Enzalutamide), Zoladex (Gosereliin Acetate), Zytiga (Abiraterone Acetate). It is understood that the chimeric antigen receptors of the present invention can also be used in combination with a medicament used to treat hormone sensitive forms of prostate cancer like for example Leuprolide Acetate.

The preferred embodiments of the present invention are further described and illustrated in the Figures and Examples of the present application. In particular, the Figures show the results of the experiments as follows:
In the Figures and in the experiments the following abbreviations were used:

| **Abbreviation** | **Explanation** |
|---|---|
| 4-1BB | tumor necrosis factor receptor superfamily member 9 |
| BLI | bioluminescence imaging |
| bw | body weight |
| C4-2 | PSMA positive prostate cancer cell line |
| CAR | Chimeric Antigen Receptor |
| CAR28 | anti-PSMA CAR with CD28 co-stimulatory domain |
| CAR41 | anti-PSMA CAR with 4-1 BB co-stimulatory domain |
| CD28 | cluster of differentiation 28 |
| CD3ζ | CD3 zeta region |
| CD45RA | cluster of differentiation 45 isoform RA |
| CD62L | cluster of differentiation 62, L-selectin |
| CR | complete remission |
| DOC | docetaxel |
| DU 145 | PSMA negative prostate cancer cell line |
| EFS | Short version of the elongation factor alpha gene promoter |
| ELISA | enzyme-linked immunosorbent assay |
| i.v | intravenously |
| i.p. | intraperitoneally |
| s.c. | subcutaneously |
| Gr.A | Granzyme A |
| Gr.B | Granzyme B. |
| IFN-g | Interferon-gamma |
| LTR | long terminal repeat of retroviral vector |
| PR | partial remission |
| R | R region of the retroviral long terminal repeat |
| scFv | single chain variable fragment |
| SD | standard deviation |
| Tcm | T cell central memory |
| Teff | T cell effector |
| Tem | T cell effector memory |
| Tn, scm | T cell naive or T stem cell memory |
| U5 | U5 region of the retroviral lonq terminal repeat |
| UT | untransduced T cell |
| WPRE | woodchuck hepatitis virus post-transcriptional regulatory element |
| XTT | sodium 3'-[1-(phenylaminocarbonyl)-3,4-tetrazolium]-bis(4-methoxy-6-nitro) benzene sulfonic acid hydrate |
| ΔU3 | deletion in the U3 region in the retroviral long terminal repeat |

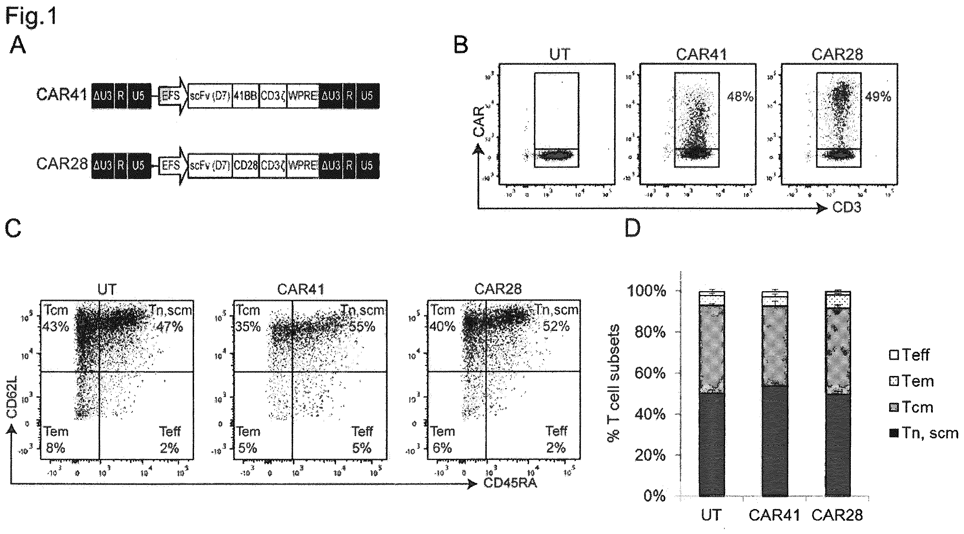

**Figure 1****. Generation and quality assessment of PSMA targeting CAR T cells.**
   **(A)** Schematic of self-inactivating gamma-retroviral vectors to express 2^{nd} generation anti-PSMA CARs. CAR expression is driven by an EFS promoter. The CAR consists of a single chain variable fragment D7 (derived from 3F/11 murine monoclonal antibody against PSMA), a hinge region (not shown), a transmembrane domain, a costimulatory domain either derived from 4-1BB (CAR41) or CD28 (CAR28), and an intracellular signaling domain derived from CD3ζ chain. The hinge region, which is included in both CARs, is derived from human IgG and provides a physical spacer between the D7-scFv and the transmembrane domain for optimal target recognition.
   **(B)** CAR expression. T cells were activated for 2-3 days with anti-CD2/3/28 antibodies prior to retrovirus transduction. Following viral transduction, T cells were expanded for 8-9 days before cells were harvested and stained with anti-human IgG antibody (CAR) and CD3 to evaluate the transduction efficiencies.
   **(C)** Qualitative CAR T cell phenotype characterization. T cell subsets were evaluated by assessing the expression of CD62L and CD45RA, pre-gated on CAR-CD3+ for the UT (not shown) or CAR+CD3+ for both types of CAR T cells (not shown).
   **(D)** Qualitative assessment of CAR T cell phenotype. Shown are the average percentages of the different T cell subsets from at least three independent experiments. PSMA, prostate specific membrane antigen; scFv, single chain variable fragment; EFS, elongation factor 1 alpha short promoter; Tn/Tscm, T cell naïve or T stem cell memory; Tcm, T cell central memory; Tem, T cell effector memory; Teff, T cell effector.
   This characterization of the CAR T cell phenotype confirms that the chosen CAR design enables the generation of high-quality of CAR T cell products with a minimal amount of terminally differentiated T cell subsets.

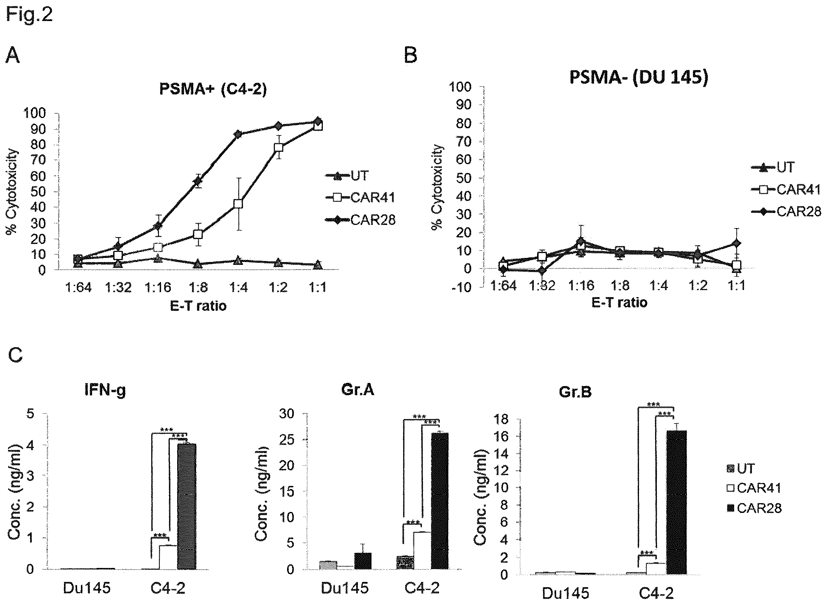

**Figure 2****. Cytotoxicity and cytokine release of PSMA-CAR T cells.**
   **(A)** Cytotoxicity profile of CAR T cells. CAR T cells were co-cultured for 48 hours at different effector to target (E:T) ratios, with either antigen positive C4-2 tumor cells or
   **(B)** Antigen negative DU 145 tumor cells Cytotoxicity was measured by an XTT ELISA based colorimetric assay (% cytotoxicity = 100 - % viability).
   A comparison of the results as presented in Figure 2A and Figure 2B shows that the T cells transfected with the CAR, according to the invention, have a cytotoxic activity only with the cell line expressing the PSMA antigen since in panel A a PSMA expressing cell line (C4-2) was used. When the cell line does not express PSMA, as for example cell line DU 145, no cytotoxicity can be seen with the CAR T cells (Figure 2B). Furthermore, the CAR28 and CAR41 cells of this invention showed superior cytotoxicity as compared to previously published data (PMID 16204083, PMID: 18026115, PMID: 19773745, PMID: 25358763, PMID: 23242161, PMID: 4174378, PMID: 25279468): up to 90% of PSMA expressing cells were eliminated (Figure 2A), using a low E:T ratio of 1:1. The observed high in vitro cytotoxicity with a low E:T ratio of 1:1 is unique and was not described in any of the available PSMA-CARs so far, indicating the superior performance our CAR T cells based on the scFv D7.
   **(C)** Quantification of pro-inflammatory cytokine release upon antigen stimulation. CAR T cells were stimulated with either PSMA-positive C4-2 cells or PSMA-negative DU 145 cells for 48 hours. Cytokines in supernatant were quantified by flow cytometry based cytokine-bead assay. Statistical significant differences are indicated by ***(P<0.001; n=3). UT; untransduced T cells, IFN-g; interferon gamma, Gr.A; Granzyme A, Gr.B; Granzyme B.
   It can be seen from Figure 2C that pro-inflammatory cytokine release occurred only when CAR T cells were co-cultured with cell lines expressing PSMA (cell line: C4-2). CAR28 were activated to a higher extend than CAR41 cells.

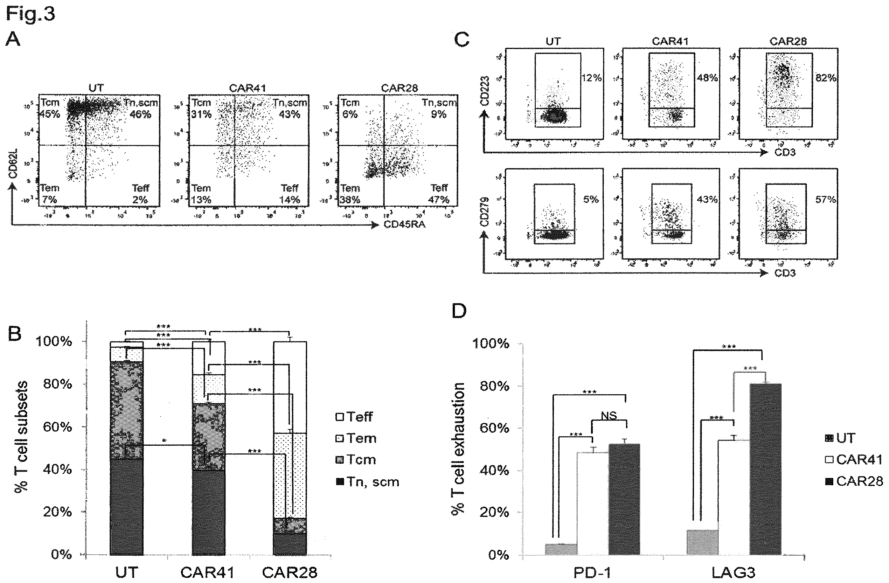

**Figure 3****. CAR T cell phenotype and exhaustion profiles upon antigen specific stimulation.**
   **(A)** CAR T cell phenotype. CAR T cells were stimulated with PSMA-positive C4-2 tumor cells for 24 hours at a 1:1 effector-to-target (E:T) ratio before T cell phenotype profile was assessed based on the expression of CD62L and CD45RA. Cells were pre-gated on either CAR-CD3+ for UT control (not shown) or CAR+CD3+ for both types of CAR T cells (not shown).
   **(B)** Quantitative assessment of CAR T cell phenotype. Shown are the average percentages of the different T cell subsets from three independent experiments. Statistical significant differences are indicated by ***(P<0.001) or *(P<0.05).
   **(C)** T cell exhaustion profile. CAR T cells were stimulated with PSMA-positive tumor cells (C4-2) for 24 hours before T cell exhaustion profile was assessed by measuring the expression of CD223 (LAG-3) and CD279 (PD-1). Cells were pre-gated on CAR-CD3+ for UT control (not shown) or CAR+CD3+ for both types of CAR T cells (not shown).
   **(D)** Quantitative assessment of CAR T cell exhaustion profiles. Shown are the average percentages of PD-1 or LAG-3 positive cells (three independent experiments). Statistical significant differences are indicated by ***(P<0.001). NS, not significant; UT, untransduced T cells; Tn/Tscm, T cell naive or T stem cell memory; Tcm, T cell central memory; Tem, T cell effector memory; Teff, T cell effector; LAG-3, lymphocyte activation gene 3; PD-1, programmed cell death protein 1.
   This set of experiments addressed the T cell phenotype and the exhaustion profiles of the CAR41 and CAR28 T cells upon antigen-specific stimulation. As seen in panels 3A and 3B, in contrast to CAR28 cells, a high percentage of CAR41 cells preserved an undifferentiated T cell phenotype upon antigen-specific stimulation. Furthermore, the CAR41 cells were less sensitive to exhaustion as compared to CAR28 T cells upon antigen-specific differentiation (Figures 3C and 3D). These features support different clinical scenarios upon administration in vivo. The high percentage of CAR41 cells with a memory T cell phenotype in combination with less exhaustion is expected to be beneficial for long-term clinical application, e.g. to eradicate metastasis and/or to prevent tumor relapse.

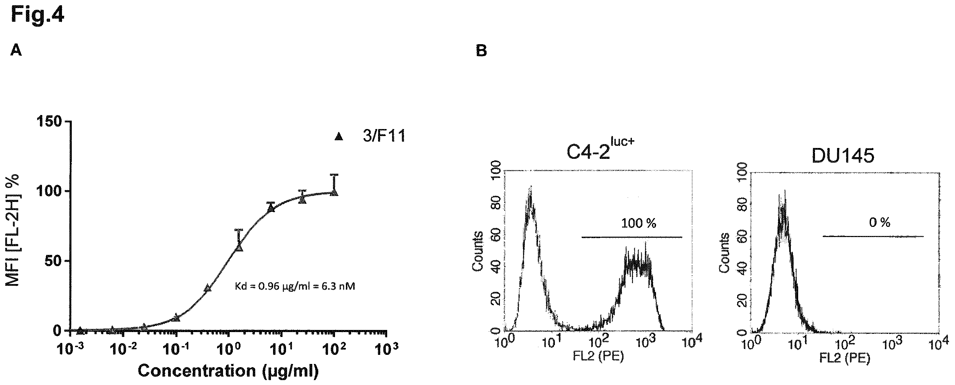

**Fig. 4****: PSMA expression of the prostate cancer target cell line C4-2^{luc+}.**
   For in vivo bioluminescence imaging, PSMA-positive prostate cancer C4-2 cells were transduced with a lentiviral vector encoding firefly luciferase, a neomycin resistance gene and green-fluorescent protein (GFP). The transduced cell line was named C4-2^{luc+}.
   **(A)** Binding of the anti-PSMA mAb 3/F11 to C4-2^{luc+} cells as determined by flow cytometry.
   **(B)** Binding at a saturation concentration of 25 µg/ml 3/F11 to C4-2^{luc+} and PSMA-negative DU 145 cells as determined by flow cytometry.
   Binding of the anti-PSMA mAb 3/F11, the parental mAb of the scFv D7, verified the PSMA expression on the surface of all C4-2^{luc+} prostate cancer target cells, while DU 145 prostate cancer control cells were shown to be PSMA negative.

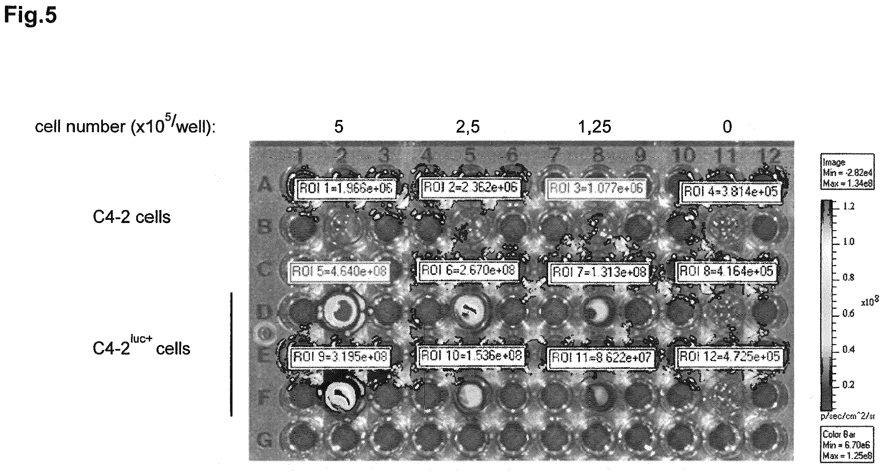

**Fig. 5****: Bioluminescence of C4-2^{luc+} cells as determined by bioluminescence imaging (BLI) with the Living imaging system IVIS 200 (Xenogen VivoVision).**
   The luminescence of the C4-2^{luc+} target cells was tested using the In Vivo Imaging System IVIS 200 (Xenogen VivoVision) after incubation with luciferin. Compared to the non-transduced C4-2 cells, the C4-2^{luc+} cells showed bioluminescence, with an intensity (photons/sec/cm²) that was linear to the cell number. Thus, these cells were suitable for their use in the mouse tumor xenograft model with tumor detection by bioluminescence imaging (BLI). ROI, region of interest.

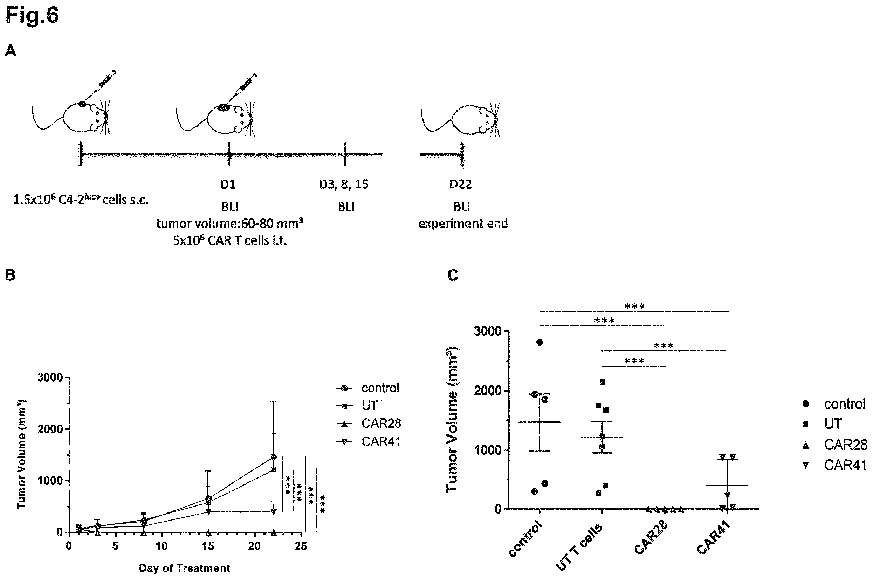

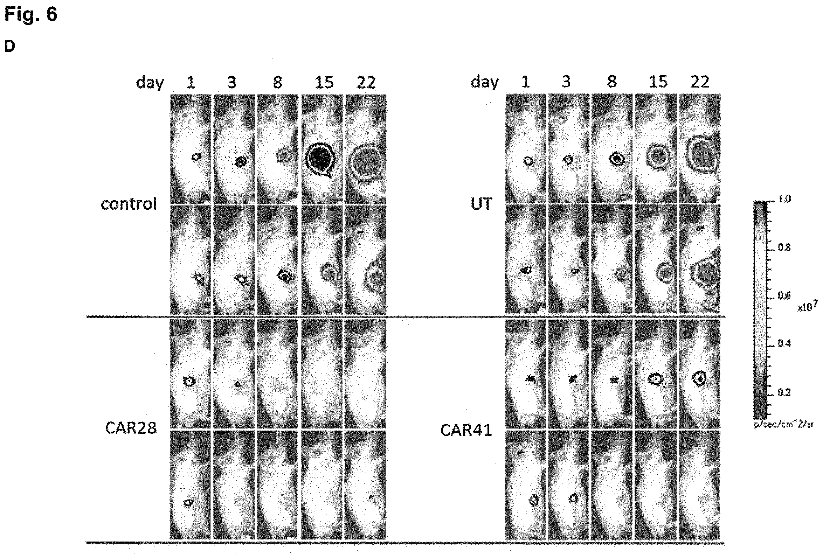

**Fig. 6** **Intratumoral CAR T cell therapy**
   **(A)** Schematic of intratumoral CAR T cell therapy. Tumor engraftment was established in animals by subcutaneous injection of 1.5x10⁶ cells. When the growing solid C4-2^{luc+} tumors have reached 60-80 mm³ volume, CAR T cells were intratumorally injected with single doses of 5x10⁶ CAR28 (n=5) or CAR41 T cells (n=5) at day 1 of treatment. Control mice were injected with the same number of UT cells (n=7) or left untreated (control, n=6). Tumors were visualized via bioluminescence imaging (BLI) until the end of treatment at day 22. D, day; i.t, intratumoral; s.c, subcutaneous.
   **(B)** Antitumor activity of CAR28 and CAR41 T cells after intratumoral injection. Animals with subcutaneously growing solid C4-2^{luc+} tumors (60-80 mm³ volume) were intratumorally injected with single doses of 5x10⁶ CAR28 (n=5) or CAR41 T cells (n=5) on day 1 of treatment. Control mice were injected with the same number of UT cells (n=7) or left untreated (control, n=6). Tumors were visualized via BLI until the end of treatment on day 22. Tumor volumes, represented as Mean ± SD were calculated from the BLI images using the formula Vol = d²xD/2, where d was the small diameter and D was the large diameter of the tumor. Statistical significant differences are indicated by ***p<0.001.
   **(C)** Antitumor activity of intratumorally injected CAR28 and CAR41 T cells at the end of treatment (day 22). Tumor volumes were calculated from BLI images as described above. Tumor volumes are shown as Mean ± SD. Statistical significant differences are indicated by ***p<0.001. Since 1/6 mice of the control group developed an ulcerating tumor, this mouse had to be euthanized on day 15 and was not included into the statistical analysis of day 22.
   **(D)** Representative bioluminescence images of mice intratumorally treated on day 1 of treatment with single injections of CAR28 or CAR41 T cells compared to untreated animals (control) or animals treated with UT T cells. Two mice representing animals with large or small tumors of each treatment group are shown.
   Intratumoral treatment of mice bearing solid C4-2^{luc+} tumors of 60-80 mm³ volume with only one injection of 5x10⁶ CAR28 T cells on day 1 of treatment led to a significant inhibition of tumor growth and to 5/5 complete tumor remissions (CR) until day 15 (Figure 6B, C and D). One animal showed a recurrence of the tumor at day 22 with a tumor end volume of 0.55 mm³ (Figure 6D, mouse pictured). For all mice of the group a mean tumor volume of 0.11 ± 0.22 mm³ was calculated for day 22 (Figure 6B and C). Mice intratumorally treated with CAR41 T cells showed a different response. 1/5 CR and 1/5 partial remission (PR) were noted leading to a statistically significant inhibition of tumor growth with a mean tumor end volume of 397.3 ± 393.6 mm³ on day 22 (Figure 6B and C). In contrast, all mice of the UT and control groups showed tumor progression leading to tumor end volumes of 1469.4 ± 961.5 mm³ and 1289.1 ± 636.8 mm³, respectively (Figure 6B and C).

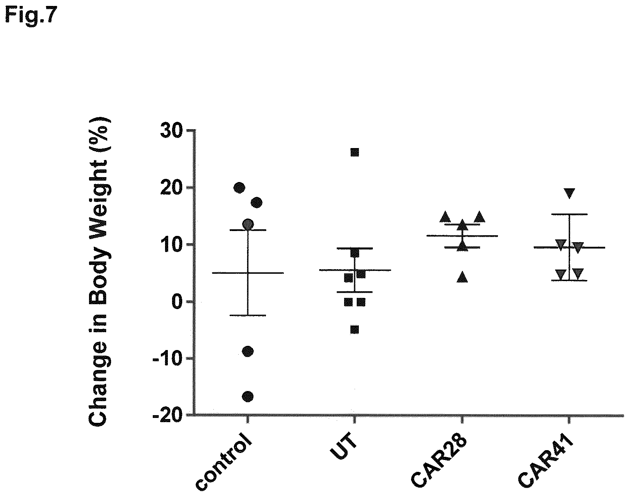

**Fig. 7****: Changes in body weight of intratumorally treated mice at the end of treatment (day 22).**
   According to animal protection guidelines, a decrease in body weight is an essential sign of toxicity during treatment. No animal showed a critical decrease of its initial body weight of more than 20%. This evidences that the intratumoral treatment was tolerated well without apparent signs of unspecific toxicity.

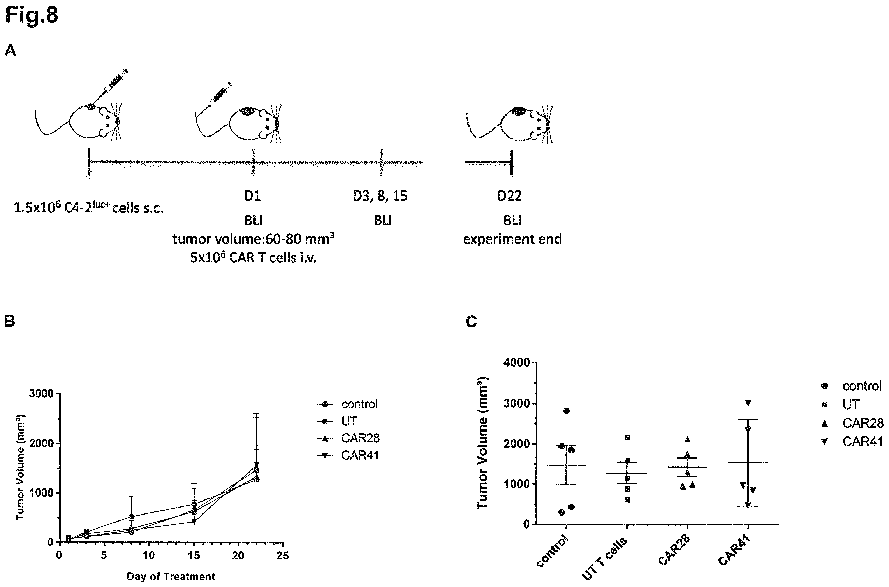

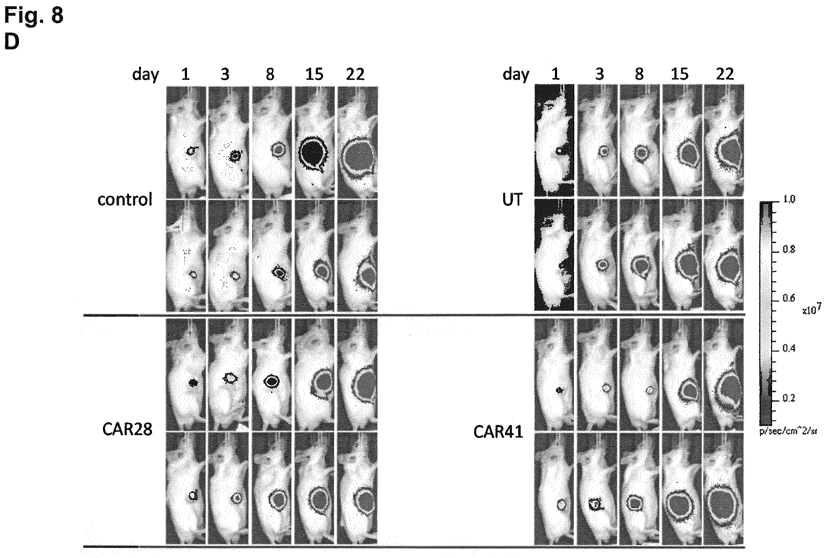

**Fig. 8****: Intravenous CAR T cell therapy.**
   **(A)** Schematic of intravenous CAR T cell therapy. Tumor engraftment was established in animals by subcutaneous injection of 1.5X10⁶ cells. When the growing solid C4-2^{luc+} tumors have reached 60-80 mm³ volume, CAR T cells were intravenously injected with single doses of 5x10⁶ CAR28 (n=5) or CAR41 T cells (n=5) on day 1 of treatment. Control mice were injected with the same number of UT cells (n=7) or left untreated (control, n=6). Tumors were visualized via BLI until the end of treatment on day 22. D, day; i.v, intravenous; s.c, subcutaneous.
   **(B)** Antitumor activity of CAR28 and CAR41 T cells after intravenous injection. Animals with subcutaneously growing solid C4-2^{luc+} tumors (60-80 mm³ volume) were intravenously injected with single doses of 5x10⁶ CAR28 (n=5) or CAR41 T cells (n=5) at day 1 of treatment. Control mice were injected with the same number of UT cells (n=5) or left untreated (control, n=6). Tumors were visualized via bioluminescence imaging until the end of treatment on day 22. Tumor volumes, represented as Mean ± SD were calculated from the BLI images using the formula Vol = d²xD/2, where d was the small diameter and D was the large diameter of the tumor.
   **(C)** Antitumor activity of intravenously injected CAR28 and CAR41 T cells at the end of treatment (day 22). Tumor volumes were calculated from BLI images as described above. Tumor volumes are shown as Mean ± SD.
   **(D)** Representative bioluminescence images of mice intravenously injected with single doses of CAR28 or CAR41 T cells. Two mice representing animals with large or small tumors of each treatment group are shown.
   No inhibition of tumor growth could be measured after single intravenous injections of CAR28 or CAR41 T cells into mice bearing solid tumors with volumes of 60-80 mm³ (Figure 8B and C). As demonstrated in Figure 8C +D, animals of all treatment groups showed comparable tumor growth leading to tumor end volumes on day 22 of 1469.4 ± 961.4 mm³ (control), 1275.7 ± 544.5 mm³ (UT), 1427.3 ± 448.5 mm³ (CAR28), and 1529.3 ± 971.0 mm³ (CAR41).

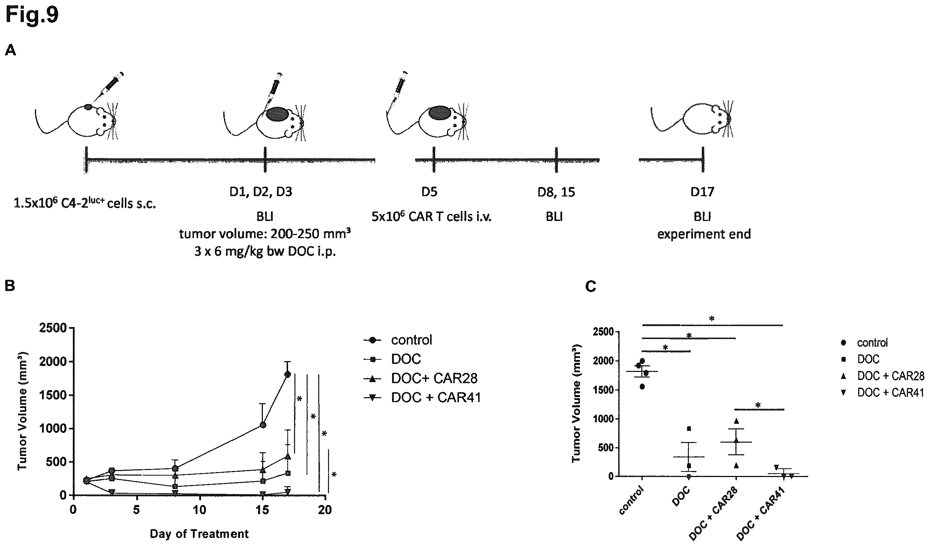

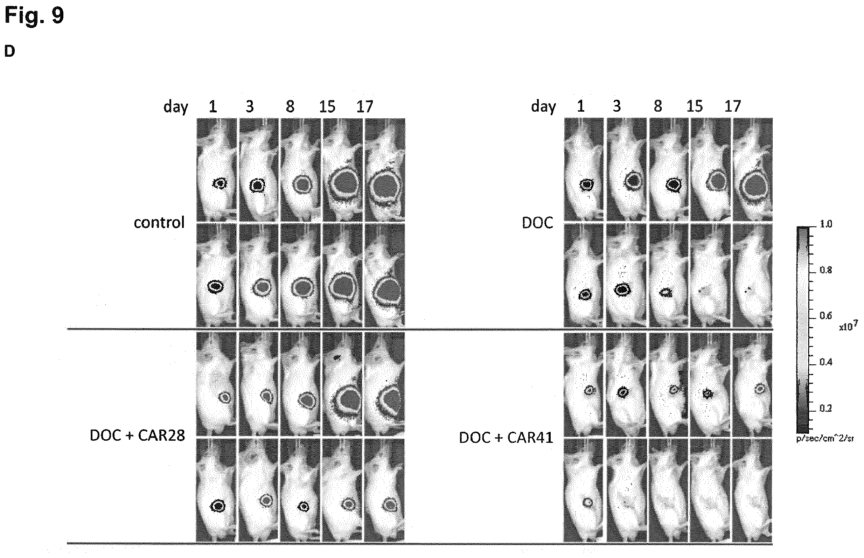

**Fig. 9****: Combination therapy**
   **(A)** Schematic of combined chemotherapy with intravenous CAR T cell therapy. Tumor engraftment was established in animals by subcutaneous injection of 1.5x10⁶ cells. Chemotherapy had started at day 1 of the treatment for 3 days by intraperitoneal injection of docetaxel (DOC, 6 mg/kg bw). 48 hours after the last chemotherapy cycle, mice were injected intravenously with single doses of 5x10⁶ CAR28 (n=3) or CAR41 T cells (n=3). Control mice were injected with docetaxel alone (n=3) or left untreated (control, n=4). Tumors were visualized via BLI until the end of treatment at day 17. D, day; i.v, intravenous injection; i.p, intraperitoneal; s.c, subcutaneous.
   **(B)** Antitumor activity of CAR28 and CAR41 T cells in combination with docetaxel chemotherapy. Mice with subcutaneously growing C4-2^{luc+} tumors (200-250 mm³) were intraperitoneally injected with docetaxel (DOC; 6 mg/kg bw) on days 1, 2, and 3 of treatment. 48 hours later mice were additionally injected with a single dose of 5x10⁶ CAR28 (n=3) or CAR41 T cells (n=3) intravenously. Tumor growth was monitored by BLI. Compared to the untreated control (n=4), DOC treatment led to a significant inhibition of tumor growth, which was enhanced by the addition of CAR28 or CAR41 T cells. 4/4 mice of the control group showed growing tumors during treatment, whereas in the DOC group 2/3 mice showed a PR. In the DOC + CAR28 group 1/3 animals showed a PR. In the DOC + CAR41 group 2/3 mice showed a CR and 1/3 mice a PR. A statistically significant difference between the DOC + CAR28 and DOC + CAR41 groups demonstrated the superior effects of the CAR41 T cells in this treatment scheme. Statistical significant differences are indicated by *p<0.05.
   **(C)** Antitumor activity of CAR28 and CAR41 T cells in combination with docetaxel chemotherapy on day 17 of treatment. At the end of treatment on day 17 the mean tumor volume of mice of the control group was determined with 1817.5 ± 165.5 mm³. The DOC group and the DOC + CAR28 group exhibited significantly lower volumes of 338.3 ± 355.6 mm³ and of 559.9 ± 317.2 mm³, respectively. Animals of the DOC + CAR41 group only had a mean tumor volume of 50.2 ± 71.1 mm³. Statistical significant differences are indicated by *p<0.05.
   **(D)** Representative bioluminescence images of mice pretreated with DOC and intravenously injected with single doses of CAR28 or CAR41 T cells. Two mice representing animals with large or small tumors of each treatment group are shown.
   Taken together, pretreatment of tumors with docetaxel chemotherapy led to an antitumor activity of intravenously applicated CAR41 T cells.
**Figure 10****: Sequences**
   Figure 10 shows the amino acid sequence ("Frame1") of the antigen-binding construct D7 which is derived from mice. Moreover, the coding nucleic acid sequence and the complementary strand thereof are provided. The CDR H1-H3 and CDR L1-L3 nucleic acid and amino acid sequences are marked with grey arrows.
**Figure 11****: Potential mutations in humanized sequences**
   In Figure 11 the CDRs are shown. Usually the CDR-H1, CDR-H3 and CDR-L3 are without any amendments. It is possible, however, to replace one amino acid in CDR-L2, up to two amino acids in CDR-L1 and up to six amino acids in CDR-H3. The amino acids which can be replaced are designated as X1-X9 with the meaning shown in the legend to Figure 11.

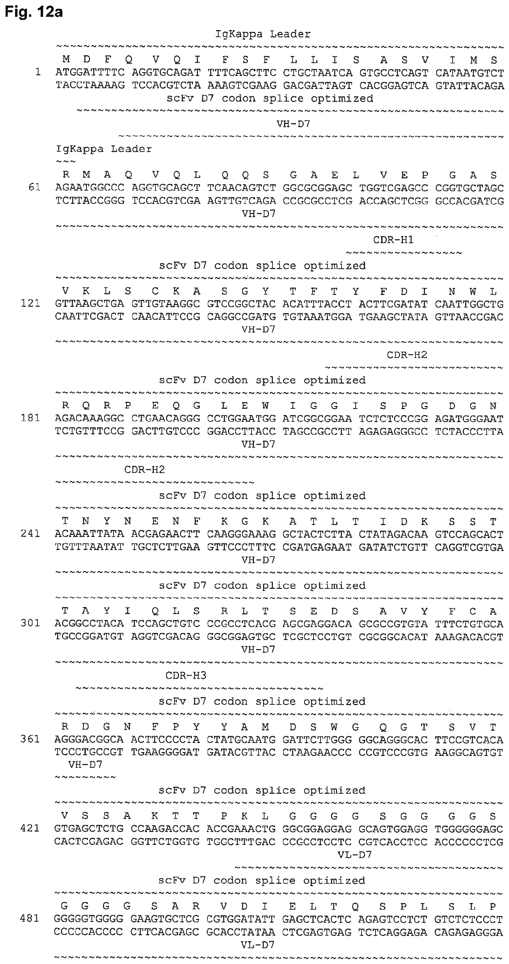

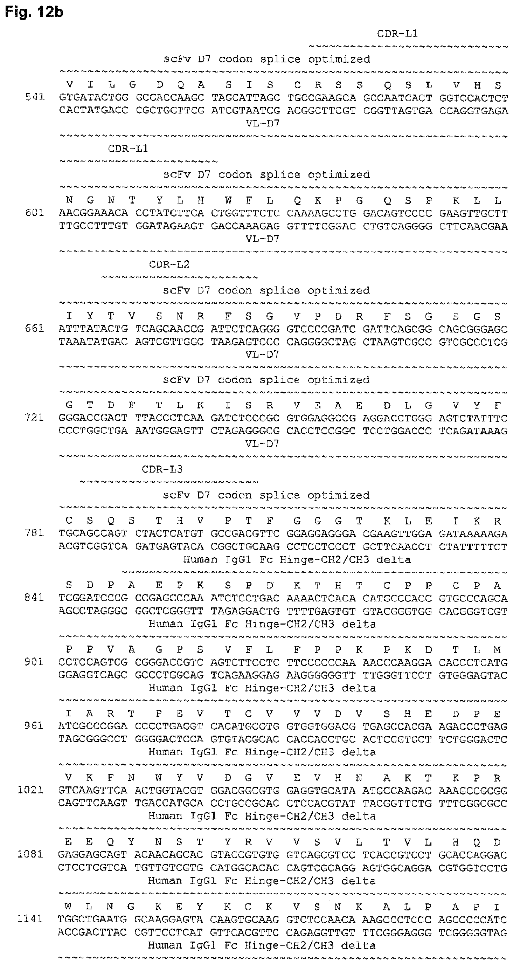

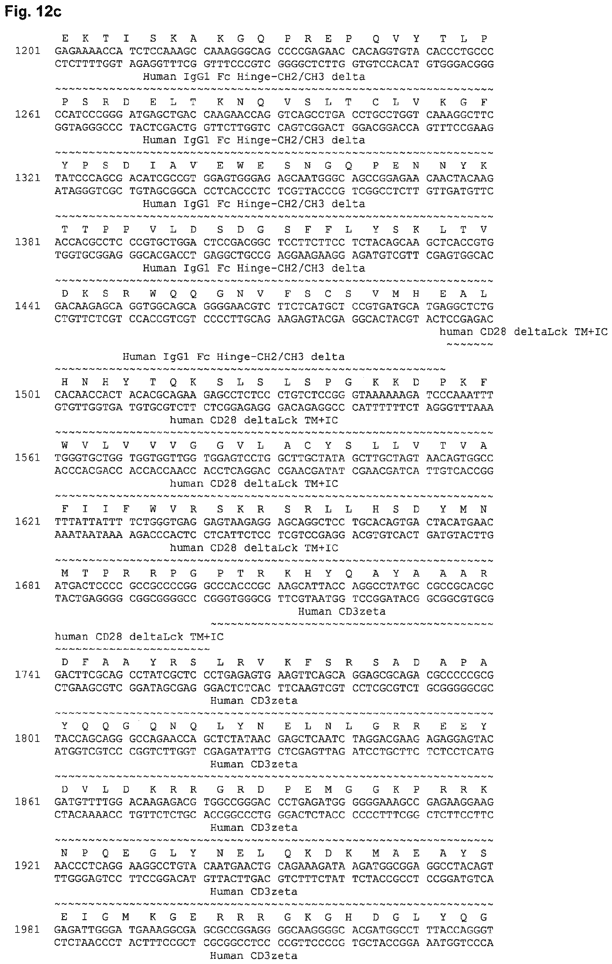

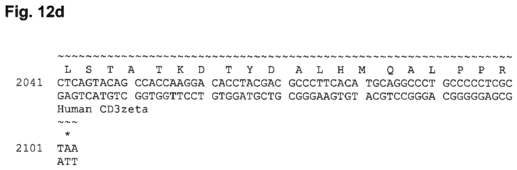

**Figure 12****: Sequences of construct CAR28**
   Figures 12a-12d show the sequences of the construct designated as CAR-CD28.

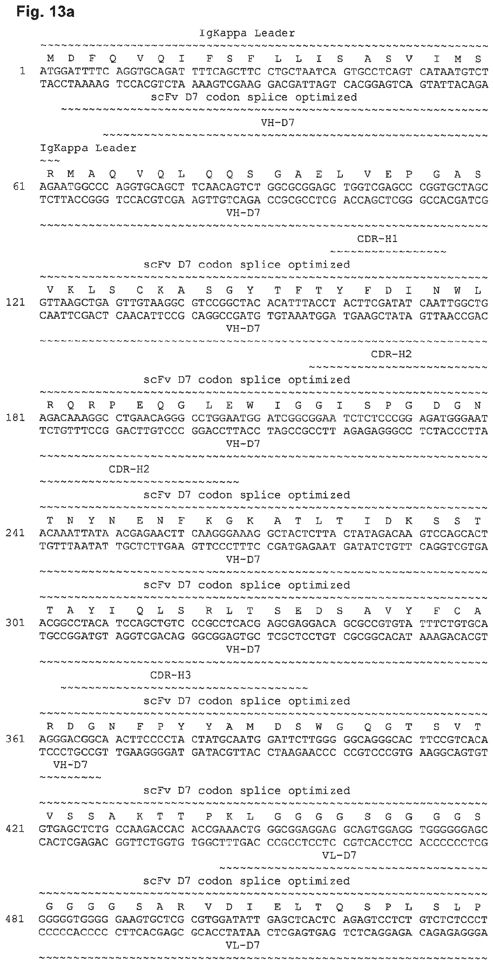

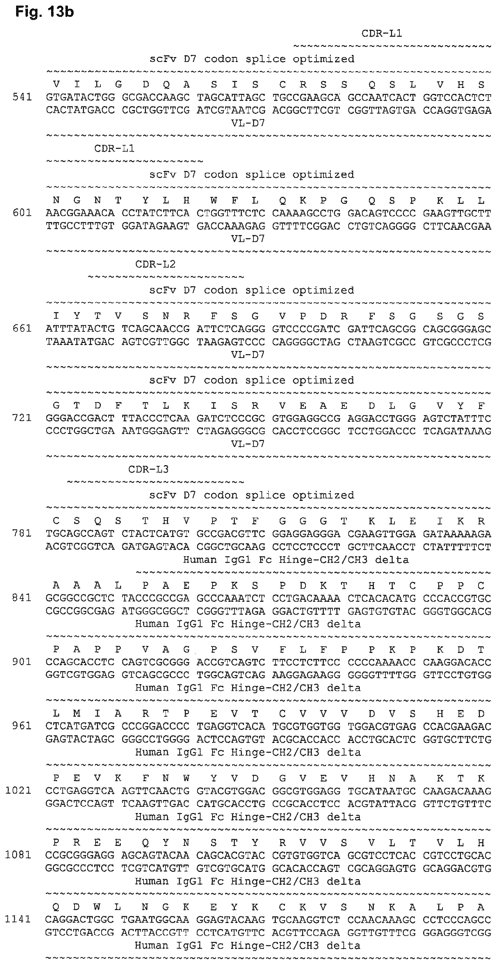

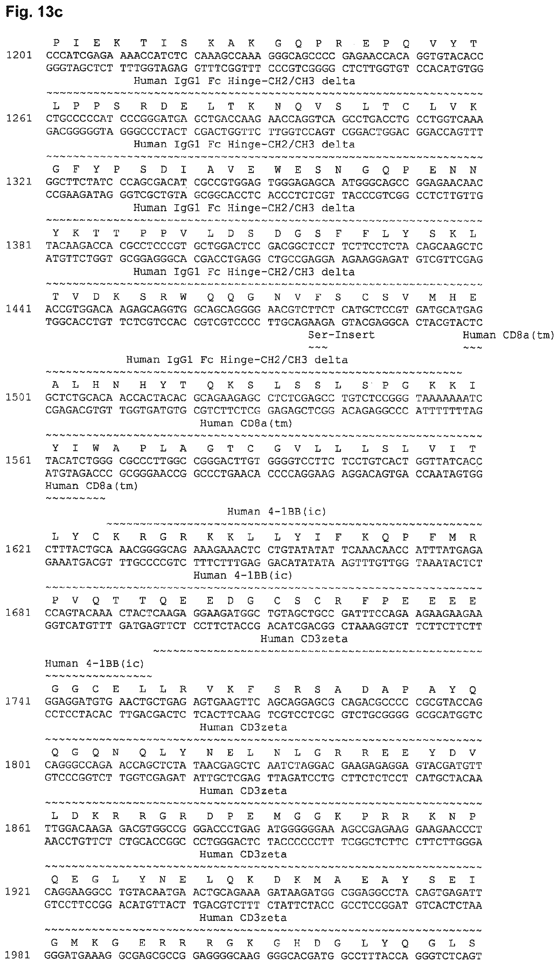

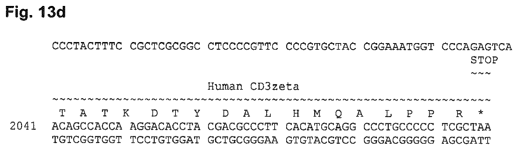

**Figure 13****: Sequence of construct CAR41**
   The sequence of the construct CAR-4-1BB is shown in Figures 13a-13d.

It should be noted that in Figures 10-13 the sequence information is provided together with an information which function the relevant parts of the sequences have. Therefore, it is understood that the person skilled in the art derives the information in the best manner from the sequences provided. Sequences 10-13 disclose preferred embodiments of the invention.

The results of the experiments shown in the Figures can be interpreted as follows:
In general, CARs are composed of an extracellular domain containing the antigen recognizing scFv, a hinge region, a transmembrane region, and one or more intracellular signaling domains that activate the T cell, including the CD3ζ chain. In 2^{nd} or 3^{rd} generation CARs, co-stimulatory domains, usually derived from CD28, 4-1BB, OX40, CD27 and/or ICOS are included (**Figure 1A**). For the treatment of prostate cancer, many attempts had utilized CARs targeting PSMA epitopes and some of these strategies have already entered clinical trials (e.g. NCT01140373, NCT01929239). However, the potency of these CARs seems to be rather low both *in vitro* and *in vivo.* In particular, early studies based on 1^{st} generation CARs based on the anti-PSMA scFv 3D8 or J591 (known as Pzl), have shown low potency of the resulting CAR T cells, as indicated by the need of having to employ high effector to target (E:T) ratios up to 100:1 to eliminate the tumor cells *in vitro.* The *in vitro* potency improved when 2^{nd} or 3^{rd} generation CARs based on either D2B or J591 derived scFvs were used. However, the potency of these CAR T cells remained low in xenotransplantion tumor mouse models, as indicated by the fact that the these PSMA targeting CAR T cells were only able to suppress tumor growth but not to eliminate the tumors *in vivo,* although very high CAR T cell doses, sometimes up to 20x10⁶ CAR T cells, or multiple infusions were applied (PMID 16204083, PMID: 18026115, PMID: 19773745, PMID: 25358763, PMID: 23242161, PMID: 4174378, PMID: 25279468). In view of these mixed results with rather inefficient PSMA-CAR T cells, it was intended to generate and validate novel, more efficient PSMA targeting CARs based on the scFv D7. Two different 2^{nd} generation PSMA-CARs were designed which either harbor a CD28 (CAR28) or 4-1BB (CAR41) derived co-stimulatory domain (**Figure 1**). The results demonstrate the high effectiveness of these newly developed D7-based PSMA-targeting CAR T cells both *in vitro* and *in vivo.* The manufactured CAR T cell products contained a high percentage of undifferentiated T cells, such as a naïve T cell, T stem cell memory and central memory T cell phenotypes (**Figure 1D**). These D7-based CAR T cells completely eliminated PSMA-positive tumor cells *in vitro* at a low effector to target ratio and released the expected cytokines upon specific antigen stimulation (**Figure 2A****-C**).

As expected, CAR41 T cells maintained a more naive phenotype and less exhaustion profile upon antigen specific stimulation as compared to CAR28 (**Figure 3**).

Importantly, the D7-based CAR T cells eliminated solid PSMA-positive tumors in a mouse tumor model upon intratumoral application (**Figure 6**). It can be seen that the control (untreated) and the control with untransduced (UT) T cells developed fast growing tumors. The two embodiments of the present invention (CAR28 T cells and CAR41 T cells), however, clearly blocked the tumor growth after an intratumoral single dose application and led to PR or CR. This proves that the concept works in vivo.

An intravenous single-dose injection of the CAR T cells of the present intervention did not cause growth inhibition of PSMA-positive solid tumors (**Figure 8**). However, after chemotherapy with docetaxel, a single dose of intravenously injected CAR41 T cells led to a complete remission of large tumors (200-250 mm³) (**Figure 9**).

In summary, it has been demonstrated that the D7-based anti-PSMA CAR T cells are superior to previously published PSMA-targeting CAR T cells in view of in vitro cytotoxicity and in vivo antitumor activity. They are hence promising tools for the development of novel immunotherapies for the treatment of advanced prostate cancer.

The present invention relates therefore to chimeric antigen receptors for T cells which comprise an antigen-binding fragment which binds specifically to the PSMA antigen. The antigen-binding fragment comprises preferably a V_{H} and a V_{L} fragment which are connected with a suitable linker. Moreover, the chimeric antigen receptor comprises preferably a spacer element, a transmembrane fragment and a CDR3ζ cytoplasmic domain. Furthermore, the chimeric antigen receptor preferably comprises a fragment from the CD28 (**Fig. 12**) and/or a 4-1BB (**Fig. 13**) cytoplasmic domain.

The chimeric antigen receptors of the present invention contain preferably at least three CDRs selected from the group consisting of CDR-H1, CDR-H2, CDR-H3, CDR-L1, CDR-L2 and CDR-L3 as shown in Figure 10. The CDRs are shown by grey arrows above the amino acid sequence and the relevant nucleic acid sequence coding. In a preferred embodiment the chimeric antigen receptor comprises at least three (CDR-H1, CDR-H3, CDR-L3), preferably four (CDR-H1, CDR-H3, CDR-L2, CDR-L3), and more preferred at least five (CDR-H1, CDR-H3, CDR-L1, CDR-L2, CDR-L3) of the CDRs as shown in Figure 10 and 11, respectively.

The chimeric antigen receptors are preferably present in a humanized format. Such a humanized format can be obtained by inserting the at least three, preferably four, more preferred five or six CDRs into a suitable human antigen-binding scaffold having a high homology to the murine scaffold as shown in **Fig. 11****.**

Usually the genetic information coding for the chimeric antigen receptor is introduced with the help of a suitable vector into the target T cells. Such a vector can preferably be a lentivirus vector or a retroviral vector or a transposon or a plasmid. Alternatively, the genetic information can be introduced into the genome of the T cell in a targeted fashion with the help of designer nuclease technology, such as the CRISPR/Cas technology or the TALEN technology, as described herein.

In a further aspect the present invention relates to an in vitro method of providing T cells comprising a chimeric antigen receptor as described herein. In the first step T cells are isolated from a donor preferably by leukapheresis methods. This results in a substantial enrichment of the T cells. The T cells are then genetically modified by transfection with a suitable vector or by transduction with a viral vector containing the genetic information for the chimeric antigen receptor, respectively. Such genetically modified T cells can then be isolated and amplified whereby those T cells which do not contain the desired genetic information can be separated from the modified T cells or at least reduced. The transfected T cells can then be applied to the patient to be treated.

### Example 1

### Preparation of CAR encoding retroviral particles

HEK293T cells were cultured at 37 °C in a humidified incubator with 5% CO₂ in DMEM (Gibco, Invitrogen, Karlsruhe, Germany) supplemented with 10% fetal calf serum (Biochrom, Berlin, Germany), penicillin (100 U/ml), streptomycin (100 mg/L) and 10 mM HEPES (Sigma-Aldrich). One day prior to transfection, cells were seeded in 10 cm dishes at cell density of 5x10⁶ cells/dish. 24 h later, cells were transfected using polyethylenimine (PEI: 0.1 mg PEI/ml, Polysicence Inc., USA). Per 10 cm dish 3 µg of a plasmid encoding the VSV-G envelope, 6 µg of a gag/pol encoding plasmid, and 10 µg of a vector plasmid coding for either CAR28 or CAR41 were used. 48 and 72 h post-transfection, supernatants containing viral vectors were collected and concentrated using ultracentrifugation (WX ultra series; Thermo Scientific: 25,000 rpm for 2 hours at 4°C). Concentrated vectors were suspended in 100 µl of cold PBS and kept at -80°C until used. Biological titers of the vector preparations were determined by transducing the Jurkat T cell line, followed by staining of the transduced cells with anti-human IgG to determine CAR positive cells.

### Example 2

### Generation of PSMA-targeting CAR T cells

CAR T cells were generated from peripheral blood mononuclear cells (PBMCs). PBMCs were isolated using phase separation (Ficoll, Sigma-Aldrich) according to the manufacture's recommendation and then frozen in liquid nitrogen until used. For generation of CAR T cells, PBMCs were thawed and let to recover for 24 h in RPMI complete medium [RPMI 1640 medium (Gibco, Invitrogen, Karlsruhe, Germany) supplemented with 10% fetal calf serum (Biochrom, Berlin, Germany), penicillin (100 U/ml), streptomycin (100 mg/L) and 10 mM HEPES buffer (Sigma-Aldrich)]. Then, PBMCs were activated using anti-CD2/CD3/CD28 antibodies (Immunocult, StemCell Technologies) and cultured with RPMI complete medium supplemented with 100 U/ml of IL-2, 25U/ml of IL-7 and 50 U/ml of IL-15 (all from Miltenyi Biotech) for 2 to 3 days before transduction with gamma-retroviral constructs encoding either CAR28 or CAR41 with MOIs ranging from 50-300. Transduced cells were cultured in wells coated with poly-D-lysin (PDL, Sigma-Aldrich) containing RPMI complete medium supplemented with 5 µg/ml of protamine sulfate (Sigma-Aldrich) and 1000 U/ml of IL-2, 25 U/ml of IL-7 and 50 U/ml of IL-15. After one day, medium was changed and cells were further expanded for 8-9 days in RPMI complete medium supplemented with 100 U/ml of IL-2, 25 U/ml of IL-7 and 50 U/ml of IL-15 before being frozen in liquid nitrogen until further use.

### Example 3

### Quality assessment of CAR T cells

To monitor CAR and TCR expression by flow cytometry (FACS Canto II or Accuri, BD Biosciences), cells were stained with anti-human IgG-PE (Southern Biotech) and CD3-APC (Miltenyi Biotec), respectively. As shown in **Figure 1B****,** up to 50% transduction efficiency was achieved for both CAR28 or CAR41. For quality assessment, CAR T cells were harvested and stained with anti-human CD62L-Bv421 (BD Biosciences), anti-human CD45RA-FITC (Biolegend), anti-human CD3-APC/H7 (BD Biosciences) and anti-human IgG-PE (CAR) (Southern Biotec) at the end of the expansion period (**Figure 1C****, D**). The T cell phenotype was determined based on the expression of CD62L and CD45RA. Cells were pre-gated on CD3+/CAR- for untransduced (UT) T cells or on CD3+/CAR+ for both types of CAR T cells (**Figures 1C****, D**). Both the transduction and expansion protocol did not induce T cell differentiation, as high percentages of undifferentiated cells with naive T cell (Tn), T stem cell memory (Tscm) or central memory T cell (Tcm) phenotypes, respectively, in combination with a low fraction of terminally differentiated effector T cells (Teff) were present (**Figures 1C****, D**), indicating that the protocol allowed for the generation of high quality CAR T cell products.

### Example 4

### In vitro cytotoxicity of manufactured CAR T cells

The cytotoxic potential of the manufactured CAR T cells was determined by assessing cell viability using the XTT assay, as previously described. CAR T cells were co-cultured with either PSMA-positive C4-2 tumor cells or antigen-negative tumor control cells (Du145) in 96 well plates for 48 h at different effector to target ratios in a final volume of 200µl/well of RPMI complete medium without any cytokines. To determine cell viability as a function of metabolic activity, 100 µl/well of medium was removed and replaced with 100 µl/well of XTT solution (Sigma-Aldrich) and cells incubated at 37°C. Colorimetric changes were quantified using an ELISA reader (Infinite F50, Tecan) at 450 nm. Cytotoxicity is indicated as the percentage of dead cells, which equals 100% minus the percentage of viable cells. Viability was calculated according to the equation [OD_{E+T} - OD_{E only}]/[OD_{Tonly} - OD_{medium only}] (E, effector cells = CAR T cells; T, target cells = tumor cells). As shown in **Figure 2A****,** CAR28 T cells eliminated -90% of target cells at an effector to target (E:T) ratio of 1:4, while CAR41 T cells demonstrated comparable activity at an E:T ratio of 1:1. Both types of CAR T cells revealed some minimal alloreactivity when co-cultured with PSMA-negative tumor cells (DU 145) (**Figure 2B**). Together, the results demonstrate that both types of D7-based CAR T cells eliminate PSMA-positive target cells with high efficiency and specificity.

### Example 5

### Cytokine release by activated CAR T cells

CAR T cells were co-cultured with either PSMA-positive C4-2 tumor cells or antigen negative DU 145 tumor cells for 48 h at an effector to target ratio of 1:1 in a final volume of 200 µl/well of RPMI complete medium without any cytokines. To evaluate the cytokines that were released from the CAR T cells during this time, supernatants were collected and evaluated by a multiplexed bead-based immunoassay (cytometric bead array, CBA assay, BD Biosciences) according to the manufacturer's recommendations. Three analytes, interferon gamma (IFN-g), granzyme A (Gr.A) and granzyme B (Gr.B), were determined (**Figure 2C**). Both types of the D7-based CAR T cells secreted the measured pro-inflammatory cytokines upon antigen stimulation, although CAR41 T cells released significantly less cytokines than CAR28 T cells.

### Example 6

### Cell differentiation and exhaustion upon antigen stimulation

CAR T cells were stimulated with PSMA-positive C4-2 tumor cells for 24 h at an effector to target ratio of 1:1, before cells were harvested and stained for T cell phenotype and exhaustion profiles. Assessment of the CAR T cell phenotype upon antigen stimulation revealed that CAR41 cells were less prone to differentiate as compared to CAR28 cells, as indicated by the presence of a significantly higher number of undifferentiated T cell subtypes, such as Tn,scm and Tcm (**Figure 3A****, B**).

To monitor CAR T cell exhaustion upon specific antigen stimulation, CAR T cells were stimulated with PSMA-positive tumor cells (C4-2) for 24 h, before cells were harvested and stained with anti-human CD279-FITC (PD-1, BD Biosciences), anti-human CD223-eFluor710 (LAG-3, BD Biosciences), anti-human CD3-APC/H7 (BD Biosciences) and anti-human IgG-PE (Southern Biotec). The exhaustion profile was determined by flow cytometry (FACS Canto II) based on the expression of CD279 (PD-1) and CD223 (LAG-3). Cells were pre-gated either on CD3+/CAR- for UT T cells or CD3+/CAR+ for both types of CARs. Yet again, CAR41 cells displayed a different phenotype than CAR28 cells. The significantly reduced number of LAG-3 positive CAR41 T cells is indicative of a less exhausted T cell phenotype (**Figure 3C****, D**).

### Example 7

### PSMA expression of the prostate cancer target cells C4-2^{luc+}

The PSMA expressing, androgen-independent prostate cancer cell line C4-2 was grown in RPMI 1640 medium (Gibco, Invitrogen, Karlsruhe, Germany) supplemented with penicillin (100 U/ml), streptomycin (100 mg/L) and 10% fetal calf serum (FCS, Biochrom, Berlin, Germany) at 37 °C in a humidified atmosphere of 5% CO₂. For *in vivo* bioluminescence imaging, C4-2 cells were transduced with a lentiviral vector encoding firefly luciferase, a neomycin resistance gene, and green-fluorescent protein (GFP). The cells were named C4-2^{luc+} cells and selected with 2mg/ml of the neomycin analog Geneticin (G-418 Sulfate) as previously described. C4-2^{luc+} cells were tested for PSMA expression by flow cytometry as described previously. In brief, 2x10⁵ C4-2^{luc+} cells/well in PBS with 3% FCS and 0.1% NaN₃ were incubated with different concentrations of the anti-PSMA mAb 3/F11, the parental mAb of the scFv D7, for 1 h on ice. After washing, cells were incubated with 25 µl goat anti-mouse Ig-RPE (Becton-Dickinson, Mountain View, CA) for 40 min on ice. Cells were then washed repeatedly and suspended in 200 µl PBS containing 1 µg/ml propidium iodide, 3% FCS and 0.1% NaN₃. Analysis of stained cells was performed using a FACSCalibur flow cytometer with CellQuest Pro software (BD Biosciences, Heidelberg, Germany).

As shown in **Figure 4A****,** the mAb 3/F11 bound with a binding constant (kd), defined as the half-maximal saturation concentration, of 0.96 µg/ml (about 6.3 nM) to the C4-2^{luc+} cells. This proved the high expression of PSMA on this cell line. C4-2^{luc+} cells were shown to be PSMA positive at saturation concentrations, while DU 145 prostate cancer control cells were shown to be PSMA negative (**Figure 4B**).

### Example 8

### Bioluminescence of C4-2^{luc+} cells

Bioluminescence of the C4-2^{luc+} cells was tested with help of the *in vivo* imaging system IVIS 200 (Xenogen VivoVision). For this, cells were seeded in different numbers into 96-well plates (Nunc Delta Surface, Thermo Fisher Scientific, Roskilde, Denmark) and incubated with 40 µl luciferin/well (BioSynth AG, Staad, Switzerland) as a substrate. Luminescence was analyzed using the software Living Image 3.0 within 10-30 min (Caliper Lifesciences).

Bioluminescence Imaging (BLI) showed that there was linearity between the number of C4-2^{luc+} cells and bioluminescence (quantified as region of interest (ROI). No luminescence signal could be detected with untransduced C4-2 cells and background staining was determined with ROI of max. 2.362x10⁶ photons/sec/cm² (**Figure 5**).

### Example 9

### Treatment of C4-2^{luc+} tumor xenografts by intratumoral injection of CAR T cells

5-6 week old male SCID CB17/lcr-Prkdc scid/Crl mice (20-25 g, Janvier Labs, St Berthevin Cedex, France) were injected with 1.5x10⁶ C4-2^{luc+} cells in PBS mixed with 50% Matrigel (Collaborative Biomedical Products, Chicago, IL) s.c. into the right flank. Tumor growth was monitored by BLI. For this, 200 µl of luciferin (BioSynth AG, Staad, Switzerland) were injected i.p. into the animals and BLI was done 10-30 min after injection under anesthesia using the Living imaging system IVIS 200 (Xenogen VivoVision). Tumor volumes were calculated from BLI pictures using the software Living Image 3.0 and the formula Volume=d²xD/2, where D was the large diameter and d the small diameter of the tumor. When tumors reached volumes of about 60-80 mm³, mice were injected intratumorally with only one dose of 5x10⁶ CAR28 (n=5) or CAR41 T cells (n=5), respectively (day 1 of treatment) (**Figure 6A**). As controls, mice were injected with un-transduced T cells (UT, n=7) or left untreated (control, n=6). Tumor growth was monitored via BLI on days 1, 3, 8, 15, and 22 of treatment. Control groups (UT and untreated) showed a fast tumor growth. A reduced tumor growth was reached with the CAR41 T cells. A marked tumor regression was found in mice treated with CAR28 T cells (**Figure 6B****, C,** and **D**). Until end of treatment at day 22, all animals of the control groups showed growing tumors. According to the animal protection guidelines, one mouse of the untreated group had to be killed due to an ulcerating tumor at day 15.

At day 22, mean tumor end volumes of 1469.4 ± 961.5 mm³ (untreated control) and 1289.1 ± 636.8 mm³ (UT) were reached, respectively. In contrast, 5/5 mice treated with CAR28 T cells showed a complete tumor remission (CR) from day 8 on and 4 mice remained tumor free until the end of the experiment. In the remaining animal a small tumor was detected at day 22 and verified by histological examination with a volume of 0.55 mm³. For all mice treated with CAR28, a mean tumor volume of 0.11 ± 0.22 mm³ was calculated for day 22, which was statistically significant compared to the control groups (*p < 0.05) (**Figure 6B** and **6D****).** Mice injected intratumorally with CAR41 T cells showed a different response to the treatment. One of the five animals treated showed a CR (**Figure 6B** and **6D**). Another animal showed a PR, whereas the others had tumor progression until day 22 of treatment. In summary, a mean tumor volume in this group of 397.7 ± 393.6 mm³ was determined (**Figure 6C**).

### Example 10

### Adverse side effects

According to the animal protection guidelines, mice were observed for apparent signs of toxicity during treatment (loss of weight and appetite, changes in pelage, fever, tension, apathy, aggression, respiratory disorders, paralyses, death) and termination criteria were defined as follows: significantly reduced food intake, decrease of the initial body weight >20%, spasms, paralysis, abnormal breathing disorders, apathy, aggressiveness as a sign of severe pain, tumor diameter >20 mm, ulcerating tumor. With exception of one mouse of the untreated control group, which had to be killed at day 15 because of an ulcerating tumor, no mouse showed apparent signs of toxicity and no animal reached one or more determination criteria. **Figure 7** demonstrates that no animal showed a critical decrease of its initial body weight of >20%. Taken together, all animals have tolerated the intratumoral treatment with the anti-PSMA CAR T cells well without apparent signs of unspecific toxicity.

### Example 11

### Treatment of C4-2^{luc+} tumor xenografts by intravenous injection of CAR T cells

Mice with C4-2^{*luc*+} tumor xenografts were intravenously injected with only one dose of 5x10⁶ CAR28 (n =5), CAR41 (n=5), or UT T cells (n=5) in 100 µl of PBS, respectively (day 1 of treatment) (**Figure 8A**). Control mice (n=6) remained untreated. Tumor growth was monitored as described above. Compared to the control (tumor end volume: 1469.4 ± 961.5 mm³) and the UT group (tumor end volume: 1275.7 ± 544.5 mm³), mice injected with CAR28 T cells showed a similar tumor growth with a mean tumor volume of 1427.3 ± 448.5 mm³ at the end of treatment at day 22 (**Figure 8B** and **8C**). For animals treated with the CAR41 T cells a mean tumor volume of 1529.3 ± 971.0 mm³ was determined (**Figure 8C**). Tumor growth was not controlled in animals treated either with CAR41 or CAR28 intravenously (**Figure 8B****, C,** and **D**).

In summary, intratumoral application of PSMA CAR T cells, both CAR28 and CAR41, completely eliminated tumors *in vivo* (**Figure 6**). In contrast, intravenous injection of the CAR T cells did not lead to any antitumor activity (**Figure 8**), suggesting that the intratumoral injection allowed the CAR T cells to bypass the TME, while upon intravenous injection the CAR T cells were likely inhibited by the TME.

### Example 12

### Treatment of C4-2^{luc+} tumor xenografts by combination of chemotherapy and intravenous injection of CAR T cells

Mice with large *C4-2*^{*luc*+} tumor xenografts (200-250 mm³) were intraperitoneally injected with 3 daily cycles of docetaxel (DOC, 6 mg/kg bw on days 1, 2, and 3 of treatment) (**Figure 9A**). 48 hours after the last cycle, mice were intravenously injected with only one dose of 5x10⁶ CAR28 (n =3) CAR41 T cells (n=3), or PBS (n=3) respectively (**Figure 9A**). Control animals (n=4) remained completely untreated. Tumor growth was monitored by BLI as described above. Compared to the control group, mice injected with DOC alone and with DOC + CAR28 showed an inhibition of tumor growth (**Figure 9B**). Compared to the control group (1817.5 ± 165.5 mm³) tumor end volumes of 338.2 ± 355.6 mm³ for mice treated with DOC alone and of 599.9 ± 317.2 mm³ for mice treated with DOC + CAR28 were reached on day 17 (**Figure 9C**). In contrast, 2/3 animals treated with the DOC + CAR41 combination therapy showed CR and 1/3 mice a PR (**Figure 9B**). A mean tumor volume of 50.2 ± 71.0 mm³ was determined (**Figure 9C**).

These results indicate that the D7-CAR T cells are effective in eliminating large tumors upon a single dose administration of CAR T cells in combination with chemotherapeutic agents, such as docetaxel (**Figure 9D**). In particular, the combination of docetaxel with CAR41 T cells showed the highest antitumor activity with CR. These results are well in line with our *in vitro* results: CAR41 T cells preserved a more naive and central memory phenotype upon antigen stimulation and were less prone to exhaust in the presence of PSMA-positive tumor target cells.

## Claims

1. Chimeric antigen receptor comprising an antigen-binding fragment which binds specifically to PSMA antigen, a CD3ζ cytoplasmatic domain and a CD28 and/or a 4-1BB cytoplasmatic domain.

2. Chimeric antigen receptor according to claim 1 **characterized in that** it contains at least three CDRs selected from SEQ ID NO:2, SEQ ID NO:4 and SEQ ID NO:7.

3. Chimeric antigen receptor according to claim 1 **characterized in that** it contains at least four CDRs selected from SEQ ID NO:2, SEQ ID NO:4, SEQ ID NO:6 and SEQ ID NO:7.

4. Chimeric antigen receptor according to claim 1 **characterized in that** it contains at least five CDRs selected from SEQ ID NO:2, SEQ ID NO:4, SEQ ID NO:5, SEQ ID NO:6 and SEQ ID NO:7.

5. Chimeric antigen receptor according to claim 1 **characterized in that** it contains at least six CDRs selected from the group consisting of SEQ ID NO:2, SEQ ID NO:3, SEQ ID NO:4, SEQ ID NO:5, SEQ ID NO:6 and SEQ ID NO:7.

6. Chimeric antigen receptor according to any of claims 1-5 **characterized in that** the antigen-binding fragment is humanized.

7. Vector coding for a chimeric antigen receptor according to any of claims 1-6 **characterized in that** it contains the nucleic acid coding for the chimeric antigen receptor according to any of claims 1-6.

8. Vector according to claim 7 **characterized in that** it comprises SEQ ID NO:10 or SEQ ID NO:11 or a sequence which is identical therewith at a degree of at least 95%.

9. In vitro method of providing T cells comprising a chimeric antigen receptor according to any of claims 1-6 comprising isolating T cells from a donor by leukapheresis; transfecting/transducing the T cells with a vector according to claim 7 coding for a chimeric antigen receptor;
isolating and amplifying the transfected/transduced T cells.

10. T cell **characterized in that** it contains the genetic information coding for a chimeric antigen receptor according to any of claims 1-6.

11. Chimeric antigen receptor according to any of claims 1-6 for use in the treatment of prostate cancer and/or prostate-derived tumors.

12. Chimeric antigen receptor for use according to claim 11 **characterized in that** the prostate-derived tumor is a solid tumor expressing PSMA.

13. Chimeric antigen receptor for use according to any of claims 11 or 12 **characterized in that** it inhibits the neovascularization of solid tumors.

14. Chimeric antigen receptor for use according to any of claims 11-13 **characterized in that** it is combined with the administration of chemotherapeutically active agents.

15. Chimeric antigen receptor for use according to claim 14 **characterized in that** the chemotherapeutically active agents are selected from cytotoxic substances comprising taxol derivatives, 5-fluorouracil, cyclophosphamide, mitoxanthrione, docetaxel and/or capacitaxel.

16. Chimeric antigen receptor for use according to any of claims 11-13 **characterized in that** it is combined with the administration of a biopharmaceutical, comprising proteins, antibodies, vaccines, blood, blood components, allergenics, recombinant therapeutic proteins, gene therapies, somatic cells, tissues, and cell therapies, in particular protein kinase inhibitors, enzyme inhibitors, anti-genomic therapeutics.


SEQUENCE LISTING

<110>  University Freiburg

<120>  Chimeric antigen receptor that bind to prostate-specific membrane
        antigen

<130>  PEP 03021Uni

<160>  11

<170>  PatentIn version 3.5

<210>  1
<211>  259
<212>  PRT
<213>  artificial sequence

<220>
<223>  D 7

<400>  1

Met Ala Gln Val Gln Leu Gln Gln Ser Gly Ala Glu Leu Val Glu Pro
1               5                   10                  15

Gly Ala Ser Val Lys Leu Ser Cys Lys Ala Ser Gly Tyr Thr Phe Thr
            20                  25                  30

Tyr Phe Asp Ile Asn Trp Leu Arg Gln Arg Pro Glu Gln Gly Leu Glu
        35                  40                  45

Trp Ile Gly Gly Ile Ser Pro Gly Asp Gly Asn Thr Asn Tyr Asn Glu
    50                  55                  60

Asn Phe Lys Gly Lys Ala Thr Leu Thr Ile Asp Lys Ser Ser Thr Thr
65                  70                  75                  80

Ala Tyr Ile Gln Leu Ser Arg Leu Thr Ser Glu Asp Ser Ala Val Tyr
                85                  90                  95

Phe Cys Ala Arg Asp Gly Asn Phe Pro Tyr Tyr Ala Met Asp Ser Trp
            100                 105                 110

Gly Gln Gly Thr Ser Val Thr Val Ser Ser Ala Lys Thr Thr Pro Lys
        115                 120                 125

Leu Glu Glu Gly Glu Phe Ser Glu Ala Arg Val Asp Ile Glu Leu Thr
    130                 135                 140

Gln Ser Pro Leu Ser Leu Pro Val Ile Leu Gly Asp Gln Ala Ser Ile
145                 150                 155                 160

Ser Cys Arg Ser Ser Gln Ser Leu Val His Ser Asn Gly Asn Thr Tyr
                165                 170                 175

Leu His Trp Phe Leu Gln Lys Pro Gly Gln Ser Pro Lys Leu Leu Ile
            180                 185                 190

Tyr Thr Val Ser Asn Arg Phe Ser Gly Val Pro Asp Arg Phe Ser Gly
        195                 200                 205

Ser Gly Ser Gly Thr Asp Phe Thr Leu Lys Ile Ser Arg Val Glu Ala
    210                 215                 220

Glu Asp Leu Gly Val Tyr Phe Cys Ser Gln Ser Thr His Val Pro Thr
225                 230                 235                 240

Phe Gly Gly Gly Thr Lys Leu Glu Ile Lys Arg Ala Asp Ala Ala Ala
                245                 250                 255

Ala Gly Ser

<210>  2
<211>  5
<212>  PRT
<213>  artificial sequence

<220>
<223>  CDR-H1

<400>  2

Tyr Phe Asp Ile Asn
1               5

<210>  3
<211>  17
<212>  PRT
<213>  artificial sequence

<220>
<223>  CDR-H2

<400>  3

Gly Ile Ser Pro Gly Asp Gly Asn Thr Asn Tyr Asn Glu Asn Phe Lys
1               5                   10                  15

Gly

<210>  4
<211>  11
<212>  PRT

<213>  artificial sequence

<220>
<223>  CDR - H3

<400>  4

Asp Gly Asn Phe Pro Tyr Tyr Ala Met Asp Ser
1               5                   10


<210>  5
<211>  16
<212>  PRT
<213>  artificial sequence

<220>
<223>  CDR - L1

<400>  5

Arg Ser Ser Gln Ser Leu Val His Ser Asn Gly Asn Thr Tyr Leu His
1               5                   10                  15


<210>  6
<211>  7
<212>  PRT
<213>  artificial sequence

<220>
<223>  CDR - L2

<400>  6

Thr Val Ser Asn Arg Phe Ser
1               5


<210>  7
<211>  8
<212>  PRT
<213>  artificial sequence

<220>
<223>  CDR - L3

<400>  7

Ser Gln Ser Thr His Val Pro Thr
1               5


<210>  8
<211>  777
<212>  DNA
<213>  artificial sequence

<220>
<223>  coding region

<400>  8
atggcccagg tgcagctgca gcagtctggg gctgaactgg tagagcctgg ggcttcagtg        60

aaactgtcct gcaaggcttc tggctacacc ttcacatact ttgacataaa ctggttgaga 120

cagaggcctg aacagggact tgagtggatt ggagggattt ctcctggaga tggtaataca 180

aactacaatg agaacttcaa gggcaaggcc acactgacta tagacaaatc ctccaccaca 240

gcctacattc agctcagcag gctgacatct gaggactctg ctgtctattt ctgtgcaaga 300

gatggcaact tcccttacta tgctatggac tcatggggtc aaggaacctc agtcaccgtc 360

tcctcagcca aaacgacacc caagcttgaa gaaggtgaat tttcagaagc acgcgtagac 420

attgagctca cccaatctcc actctccctg cctgtcattc ttggagatca agcctccatc 480

tcttgcagat ctagtcagag ccttgtacac agtaatggaa acacctattt acattggttt 540

ctgcagaagc caggccagtc tccaaagctc ctgatctaca cagtttccaa ccgattttct 600

ggggtcccag acaggttcag tggcagtgga tcagggacag atttcacact caagatcagc 660

agagtggagg ctgaggatct gggagtttat ttctgctctc aaagtaccca tgttcccacg 720

ttcggagggg ggaccaagct ggaaataaaa cgggctgatg ctgcggccgc tggatcc 777

<210> 9
<211> 777
<212> DNA
<213> artificial sequence

<220>
<223> complementary strand

<400> 9
taccgggtcc acgtcgacgt cgtcagaccc cgacttgacc atctcggacc ccgaagtcac 60

tttgacagga cgttccgaag accgatgtgg aagtgtatga aactgtattt gaccaactct 120

gtctccggac ttgtccctga actcacctaa cctccctaaa gaggacctct accattatgt 180

ttgatgttac tcttgaagtt cccgttccgg tgtgactgat atctgtttag gaggtggtgt 240

cggatgtaag tcgagtcgtc cgactgtaga ctcctgagac gacagataaa gacacgttct 300

ctaccgttga agggaatgat acgatacctg agtaccccag ttccttggag tcagtggcag 360

aggagtcggt tttgctgtgg gttcgaactt cttccactta aaagtcttcg tgcgcatctg 420

taactcgagt gggttagagg tgagagggac ggacagtaag aacctctagt tcggaggtag 480

agaacgtcta gatcagtctc ggaacatgtg tcattacctt tgtggataaa tgtaaccaaa 540

gacgtcttcg gtccggtcag aggtttcgag gactagatgt gtcaaaggtt ggctaaaaga 600

ccccagggtc tgtccaagtc accgtcacct agtccctgtc taaagtgtga gttctagtcg 660

tctcacctcc gactcctaga ccctcaaata aagacgagag tttcatgggt acaagggtgc 720

aagcctcccc cctggttcga cctttatttt gcccgactac gacgccggcg acctagg 777

<210> 10

<211>  2103
<212>  DNA
<213>  artificial sequence

<220>
<223>  construct CD 28

<400>  10
atggattttc aggtgcagat tttcagcttc ctgctaatca gtgcctcagt cataatgtct      60

agaatggccc aggtgcagct tcaacagtct ggcgcggagc tggtcgagcc cggtgctagc     120

gttaagctga gttgtaaggc gtccggctac acatttacct acttcgatat caattggctg     180

agacaaaggc ctgaacaggg cctggaatgg atcggcggaa tctctcccgg agatgggaat     240

acaaattata acgagaactt caagggaaag gctactctta ctatagacaa gtccagcact     300

acggcctaca tccagctgtc ccgcctcacg agcgaggaca gcgccgtgta tttctgtgca     360

agggacggca acttccccta ctatgcaatg gattcttggg ggcagggcac ttccgtcaca     420

gtgagctctg ccaagaccac accgaaactg ggcggaggag gcagtggagg tggggggagc      480

gggggtgggg gaagtgctcg cgtggatatt gagctcactc agagtcctct gtctctccct     540

gtgatactgg gcgaccaagc tagcattagc tgccgaagca gccaatcact ggtccactct     600

aacggaaaca cctatcttca ctggtttctc caaaagcctg gacagtcccc gaagttgctt     660

atttatactg tcagcaaccg attctcaggg gtccccgatc gattcagcgg cagcgggagc     720

gggaccgact ttaccctcaa gatctcccgc gtggaggccg aggacctggg agtctatttc     780

tgcagccagt ctactcatgt gccgacgttc ggaggaggga cgaagttgga gataaaaaga     840

tcggatcccg ccgagcccaa atctcctgac aaaactcaca catgcccacc gtgcccagca     900

cctccagtcg cgggaccgtc agtcttcctc ttccccccaa aacccaagga caccctcatg     960

atcgcccgga cccctgaggt cacatgcgtg gtggtggacg tgagccacga agaccctgag    1020

gtcaagttca actggtacgt ggacggcgtg gaggtgcata atgccaagac aaagccgcgg    1080

gaggagcagt acaacagcac gtaccgtgtg gtcagcgtcc tcaccgtcct gcaccaggac    1140

tggctgaatg gcaaggagta caagtgcaag gtctccaaca aagccctccc agcccccatc    1200

gagaaaacca tctccaaagc caaagggcag ccccgagaac cacaggtgta caccctgccc    1260

ccatcccggg atgagctgac caagaaccag gtcagcctga cctgcctggt caaaggcttc    1320

tatcccagcg acatcgccgt ggagtgggag agcaatgggc agccggagaa caactacaag    1380

accacgcctc ccgtgctgga ctccgacggc tccttcttcc tctacagcaa gctcaccgtg    1440

gacaagagca ggtggcagca ggggaacgtc ttctcatgct ccgtgatgca tgaggctctg    1500

cacaaccact acacgcagaa gagcctctcc ctgtctccgg gtaaaaaaga tcccaaattt    1560

tgggtgctgg tggtggttgg tggagtcctg gcttgctata gcttgctagt aacagtggcc    1620

tttattattt tctgggtgag gagtaagagg agcaggctcc tgcacagtga ctacatgaac    1680

atgactcccc gccgccccgg gcccacccgc aagcattacc aggcctatgc cgccgcacgc 1740

gacttcgcag cctatcgctc cctgagagtg aagttcagca ggagcgcaga cgcccccgcg 1800

taccagcagg gccagaacca gctctataac gagctcaatc taggacgaag agaggagtac 1860

gatgttttgg acaagagacg tggccgggac cctgagatgg ggggaaagcc gagaaggaag 1920

aaccctcagg aaggcctgta caatgaactg cagaaagata agatggcgga ggcctacagt 1980

gagattggga tgaaaggcga gcgccggagg ggcaaggggc acgatggcct ttaccagggt 2040

ctcagtacag ccaccaagga cacctacgac gcccttcaca tgcaggccct gccccctcgc 2100

taa 2103

<210> 11
<211> 2097
<212> DNA
<213> artificial sequence

<220>
<223> 4-1 BB

<400> 11
atggattttc aggtgcagat tttcagcttc ctgctaatca gtgcctcagt cataatgtct 60

agaatggccc aggtgcagct tcaacagtct ggcgcggagc tggtcgagcc cggtgctagc 120

gttaagctga gttgtaaggc gtccggctac acatttacct acttcgatat caattggctg 180

agacaaaggc ctgaacaggg cctggaatgg atcggcggaa tctctcccgg agatgggaat 240

acaaattata acgagaactt caagggaaag gctactctta ctatagacaa gtccagcact 300

acggcctaca tccagctgtc ccgcctcacg agcgaggaca gcgccgtgta tttctgtgca 360

agggacggca acttccccta ctatgcaatg gattcttggg ggcagggcac ttccgtcaca 420

gtgagctctg ccaagaccac accgaaactg ggcggaggag gcagtggagg tggggggagc 480

gggggtgggg gaagtgctcg cgtggatatt gagctcactc agagtcctct gtctctccct 540

gtgatactgg gcgaccaagc tagcattagc tgccgaagca gccaatcact ggtccactct 600

aacggaaaca cctatcttca ctggtttctc caaaagcctg gacagtcccc gaagttgctt 660

atttatactg tcagcaaccg attctcaggg gtccccgatc gattcagcgg cagcgggagc 720

gggaccgact ttaccctcaa gatctcccgc gtggaggccg aggacctggg agtctatttc 780

tgcagccagt ctactcatgt gccgacgttc ggaggaggga cgaagttgga gataaaaaga 840

gcggccgctc tacccgccga gcccaaatct cctgacaaaa ctcacacatg cccaccgtgc 900

ccagcacctc cagtcgcggg accgtcagtc ttcctcttcc ccccaaaacc caaggacacc 960

ctcatgatcg cccggacccc tgaggtcaca tgcgtggtgg tggacgtgag ccacgaagac 1020

cctgaggtca agttcaactg gtacgtggac ggcgtggagg tgcataatgc caagacaaag 1080

ccgcgggagg agcagtacaa cagcacgtac cgtgtggtca gcgtcctcac cgtcctgcac 1140

caggactggc tgaatggcaa ggagtacaag tgcaaggtct ccaacaaagc cctcccagcc 1200

cccatcgaga aaaccatctc caaagccaaa gggcagcccc gagaaccaca ggtgtacacc 1260

ctgcccccat cccgggatga gctgaccaag aaccaggtca gcctgacctg cctggtcaaa 1320

ggcttctatc ccagcgacat cgccgtggag tgggagagca atgggcagcc ggagaacaac 1380

tacaagacca cgcctcccgt gctggactcc gacggctcct tcttcctcta cagcaagctc 1440

accgtggaca agagcaggtg gcagcagggg aacgtcttct catgctccgt gatgcatgag 1500

gctctgcaca accactacac gcagaagagc ctctcgagcc tgtctccggg taaaaaaatc 1560

tacatctggg cgcccttggc cgggacttgt ggggtccttc tcctgtcact ggttatcacc 1620

ctttactgca aacggggcag aaagaaactc ctgtatatat tcaaacaacc atttatgaga 1680

ccagtacaaa ctactcaaga ggaagatggc tgtagctgcc gatttccaga agaagaagaa 1740

ggaggatgtg aactgctgag agtgaagttc agcaggagcg cagacgcccc cgcgtaccag 1800

cagggccaga accagctcta taacgagctc aatctaggac gaagagagga gtacgatgtt 1860

ttggacaaga gacgtggccg ggaccctgag atggggggaa agccgagaag gaagaaccct 1920

caggaaggcc tgtacaatga actgcagaaa gataagatgg cggaggccta cagtgagatt 1980

gggatgaaag gcgagcgccg gaggggcaag gggcacgatg gcctttacca gggtctcagt 2040

acagccacca aggacaccta cgacgccctt cacatgcagg ccctgccccc tcgctaa 2097